# EUROPEAN PATENT APPLICATION

(11) **EP 4 349 409 A1**
(43) Date of publication of application: **10.04.2024**
(21) Application number: 22382943.3
(22) Date of filing: 06.10.2022
(51) Int. Cl.: A61P 35/00, C07D 493/04, C12N 1/14, A61K 31/353

(54) **COMPOUNDS FOR THE MANAGEMENT OF CELL STEMNESS AND INVASION IN CANCER AND RELATED DISEASES**

(71) Applicant: Instituto Biomar S.A., 24009 Armunia, León (ES)
(72) Inventor: VILLOCH FERNÁNDEZ, Javier, 24071 León (ES); MARQUÉS MARTÍNEZ, Margarita M., 24071 León (ES); MARÍN VIEIRA, Maria del Carmen, 24071 León (ES); MARTÍN LÓPEZ, Marta, 24009 León (ES); SÁNCHEZ LÓPEZ, José María, 24009 León (ES); FERNÁNDEZ MEDARDE, Antonio, 24009 León (ES); VINUESA NAVARRO, María de los Angeles, 24009 León (ES); CAÑEDO HERNÁNDEZ, María Librada, 28770 Madrid (ES)
(74) Representative: ABG Intellectual Property Law, S.L.

(57) **Abstract**

The present invention relates to compound of general formula (I): 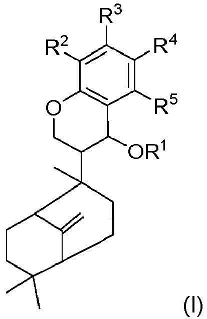 wherein the variables take the various meanings, pharmaceutical compositions containing them and their use in medicine, particularly in cancer such as Glioblastoma. The compounds have shown to be useful in therapy as modulators of TAP73 and stemness- and invasiveness-related genes. The compounds may be isolated from the fermentation broth of a fungal strain of *Chaetomium sp.,* or are derivatives thereof. The strain of *Chaetomium sp.* HT-GU-SUES-904 with accession number CECT 20840 is also claimed.

## Description

### TECHNICAL FIELD

The present invention relates to compounds, pharmaceutical compositions comprising them and their use in medicine, particularly in the management of cancer. The present invention also relates to processes for preparing such compounds and compositions.

### BACKGROUND OF THE INVENTION

Glioblastoma (GB) is the most common and fatal type of brain tumor. Clinical treatment for GB includes surgical resection followed by post-operative radiotherapy with concomitant and adjuvant temozolomide (TMZ), an oral alkylating agent (Stupp et al., 2005). However, TMZ is not effective in all patients, and treatments for grade IV gliomas remain palliative (Seymour et al., 2015). The absence of proper treatments, together with GB invasiveness and increasing incidence, have sparked the search for new therapeutic targets. One explanation for the lack of successful treatments could be the poor understanding of the cellular and molecular mechanisms governing GB initiation, progression and drug resistance, which has led to use of the wrong targets during the search for therapeutic candidates.

Cancer cells expressing stem cell markers and having the ability to self-renew, named Cancer Stem Cells (CSCs), have been identified in a variety of human cancers including high-grade gliomas (Lapidot et al., 1994; Al-Hajj et al., 2003; Singh et al., 2003; Hemmati et al., 2003). Cumulative evidence supports the idea of the existence of a small subpopulation of glioblastoma neural stem-like cells (GNSCs), with self-renewal and multipotency capacity, and characterized by the expression of neural stem cell markers like CD133+/Prominin-1. These cells would be responsible, at least in part, for glioblastoma initiation, progression, heterogeneity and increased resistance to treatments (Lapidot et al., 1994; Al-Hajj et al., 2003; Singh et al., 2003; Hemmati et al., 2003; Bao et al., 2006). These features make glioblastoma stem cells (GSCs) a very attractive model to test candidate compounds for GB treatment. Indeed, long-term expansion of neural stem cells from patient's GB samples has been an improvement over "classic" glioma cell lines, which fail to model accurately the human disease (Galli et al., 2004; Lee et al., 2006). Given the diverse spectrum of cell heterogeneity and mutational changes in GB, a major challenge is to identify a molecular targeted strategy that can efficiently disrupt growth of all subtypes of tumor cells, with a lesser effect on normal neural progenitor cell function.

The tumor protein p73 gene (*TP73* gene), which belongs to the p53 family, can give rise to different isoforms. The TA isoforms, with a full transactivation domain, and the amino-terminally truncated DNp73 isoforms (Vikhreva et al., 2018) are generated from two different promoters, P1 and P2, respectively. Due to alternative splicing at the N-terminus, the truncated isoforms ΔEx2p73, ΔEx2/3p73, and ΔN'p73 could be generated from the P1 promoter. Based on the cumulative data regarding p73 in GB, we propose the isoforms generated by the *TP73* gene, and particularly TAp73, as a specific therapeutic target. The TA and DNp73 isoforms can suffer alternative splicing at the C-terminus generating several isoforms (α, β, ε, γ, ζ, δ), which differ in their potential to activate target genes. TAp73α and TAp73β are the two main expressed isoforms in human cells, with TAp73β being the isoform with the highest level of transcriptional activity among them (Ueda et al., 1999).

The initially accepted paradigm indicates that TAp73 tumor suppressor activity relies on p53-canonical functions, like its ability to induce cell cycle arrest, apoptosis or regulation of DNA damage response (Tomasini et al., 2008; Costanzo et al., 2014; Wolfsberger et al., 2021), while the truncated forms were pro-oncogenic. However, despite the generally accepted pro-apoptotic function of TAp73, in certain contexts TAp73α can antagonize the apoptosis induced by a range of death stimuli and be overexpressed in cancers (Muppani et al., 2011). In this respect, in glioma cells, TAp73α and TAp73β may have opposite roles in regulating proliferation and apoptosis (Cheng et al., 2018). Moreover, unlike *TP53, TP73* is not mutated in tumors; on the contrary, increased TAp73α expression levels have been reported in cervical cancer (Wei et al., 2012), B-cell chronic lymphocytic leukemia (Novak et al., 2001), ovarian carcinomas (Niyazi et al., 2003), gastric adenocarcinoma (Kang et al., 2000), medulloblastoma (MB) (Zitterbart et al., 2007) and GB (Niklison-Chirou et al., 2017). Furthermore, a recent study that explores the expression of p73 isoforms across eight major cancer types (using The Cancer Genome Atlas- data) showed that TAp73 is overexpressed in breast invasive carcinoma, stomach adenocarcinoma, lung squamous cell carcinoma, colon adenocarcinoma, and esophageal carcinoma tumors (Iscan et al., 2022).

Thus, TAp73 function in tumors has been highly controversial, and a review of the literature indicates that p73 plays a dual role in cancer, acting as a tumor suppressor by regulating apoptosis in response to genotoxic stress in early tumor stages, and as a pro-oncogene by affecting the tumor microenvironment at advance stages. The experimental evidence favors the hypothesis that at the advanced stages of cancer development, tumor cells may employ p73 to repress the immune system surveillance, explaining why p73 is rarely mutated (reviewed in Rozenberg et al., 2021).

According to the latest studies, the role of p73 in the regulation of epithelial-mesenchymal transition (EMT) and invasion is also cancer type-specific, promoting invasion of GB cell lines. Indeed, TAp73α expression is frequently high in GB and MB, suggesting a pro-survival role in these tumors (Zitterbart et al., 2007). It is noteworthy that TAp73, unexpectedly, activates anabolic pathways compatible with proliferation and promotion of cancer cells (Jiang et al., 2013; Velletri et al., 2013; Amelio et al., 2014). Moreover, TAp73 cooperates with several AP-1 members to sustain cellular proliferation, suggesting that inhibition of TAp73/AP-1 cooperation may be an avenue to inhibit tumor cell growth, especially in cancers that overexpress TAp73 (Fernandez-Garcia et al., 2007; Subramanian et al., 2015). This possibility should be considered when developing new anti-cancer therapeutic strategies; thus, treatments that repress p73 isoforms could be of interest to treat p73-overexpressing tumors at late stages. On the other hand, recent reports suggest that in cancers lacking wild-type p53, ablation of E2F family members as well as p73 causes cell-cycle arrest, making these proteins potential targets of interest (Klein et al., 2021).

The role of TAp73 in brain tumors is of particular relevance. *Trp73* is highly expressed in the hippocampus, cortex, and cerebellum during embryonic stages of mouse central nervous system (CNS) development; after birth, its expression decreases and is restricted to the neural stem cell niches in the adult brain (Pozniak et al., 2002). p73 is not only essential for the embryonic development of the murine CNS (Yang et al., 2000), but also for the organization and maintenance of neural stem cells (NSC) niches (Pozniak et al., 2002; Gonzalez-Cano et al., 2016). Indeed, lack of p73 results in premature differentiation of NSC (Gonzalez-Cano et al., 2010). GSCs have similarities to fetal NSCs (Sun et al., 2008). In agreement with p73 role in NSC maintenance, most high-grade glial tumors display strong up-regulation of TAp73 (Ugur et al., 2004). Other studies have confirmed that TAp73α is the predominant isoform in MB and that its levels are higher in aggressive MB compared with either the other subgroups or normal cerebella (Niklison-Chirou et al., 2017). Those investigators demonstrated that the high levels of TAp73 sustained cell growth and proliferation via the regulation of glutamine metabolism. Some cancer cells show glutamine addiction (DeBerardinis & Cheng, 2010) and it has been proposed that targeting metabolic dependence could be a selective approach to treat cancer patients (Still & Yuneva, 2017). This tumor-suppressive effect of glutamine starvation has been shown to be an effective treatment in other cancers, including GB (Napoli & Flores, 2017; Still & Yuneva, 2017). Remarkably, down-regulation of *TP73* results in glutamine starvation of MB cells impairing their growth (Niklison-Chirou et al., 2017).

Glioblastoma cell migration, invasion and metastasis are mainly mediated through the epithelial-mesenchymal transition and the modulation of the cytoskeletal actin framework (Iwadate, 2016). Hence, targeting cell adhesion mechanisms essential for self-renewal, niche-driven GNSCs maintenance, and tumor growth could facilitate the development of more effective GB therapies (Lathia et al., 2015). In this regard, a growing body of work points to TAp73 as a central hub that orchestrates transcriptional programs involved in the control of cell junction establishment, actin and microtubule cytoskeleton dynamics and integrin associated-signaling (Wang et al., 2012; Santos Guasch et al., 2018; Xie et al., 2018; López-Ferreras et al., 2021; Maeso-Alonso et al., 2021). Several reports indicate that increase TAp73 expression confers an invasive phenotype in glioblastoma multiforme (GBM) cells, and that knocking down endogenous p73 reduces invasiveness and chemo-resistance and promotes differentiation *in vitro* (Landré et al., 2016). Moreover, TAp73 regulates integrin coding genes (Landré et al., 2016; Xie et al., 2018; Beeler et al., 2019), which have been associated to cancer stem cell maintenance and tumor formation capacity (Lathia et al., 2010; Ma et al., 2019). Another report indicates that TAp73 positively regulates the growth of cancer stem-like cells and that TAp73 knockdown in CD133+-patient-derived brain tumor-initiating cells strongly decreased their self-renewal capacity and the expression of pro-survival factors (Sharif et al., 2019), altogether strongly backing up TAp73 role in sustaining GB growth and GSC maintenance and invasiveness. All considered, we hypothesized that downregulation of the isoforms generated by the *TP73* gene promoter, and particularly TAp73, could be an interesting therapeutic strategy in several cancers, including GBM, as well as other diseases in which p73 deregulation is involved.

Thus, there is a need of new compounds able to modulate the expression of p73, which can be used as therapeutic agents in diseases associated with deregulation of p73 such as cancer and related diseases.

### BRIEF DESCRIPTION OF THE INVENTION

The present invention solves the aforementioned need by the provision of new compounds able to repress the transcriptional activity of P1-TP73 gene promoter, reducing the expression of P1-driven p73 isoforms such as, TAp73, ΔEx2p73, ΔEx2/3p73, and ΔN'p73. Further, the proposed compounds are also useful to downregulate stemness- and invasiveness-related genes. Advantageously, it has been found *inter alia* that the compounds of the invention may be more effective against glioblastoma neural stem cells (GNSC) than first line drugs Temozolomide (TMZ) and Carmustine (BCNU) reducing their stemness and invasiveness.

In one aspect, the present invention is directed to a compound of general formula (I): 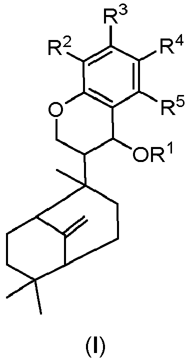 wherein
R¹ is hydrogen or a hydroxyl protecting group:
any two adjacent R², R³, R⁴, and R⁵, together with the atoms they attach to, form a 2,5-dihydrofuran ring which can be substituted with one or more substituents selected from the group consisting of =O and -OR⁷, wherein R⁷ is hydrogen or a hydroxyl protecting group; and
the other two R², R³, R⁴, or R⁵ are independently selected from the group consisting of hydrogen, C₁-C₆ alkyl, -CHO, -COOH and -OR⁶, wherein R⁶ is hydrogen or a hydroxyl protecting group;
or a pharmaceutically acceptable salt, stereoisomer or solvate thereof;
with the proviso that the compound is not

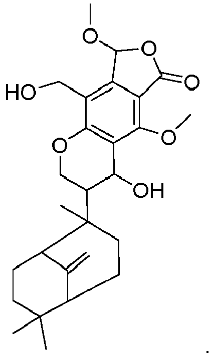

Another aspect of this invention refers to a medicament or pharmaceutical composition comprising a compound of formula (I) as defined above, or a pharmaceutically acceptable salt, stereoisomer or solvate thereof, and a pharmaceutically acceptable excipient.

Another aspect of this invention refers to a compound of formula (I) as defined above, or a pharmaceutically acceptable salt, stereoisomer or solvate thereof, for use in medicine, particularly for the treatment and/or prophylaxis of diseases associated with deregulation of p73 as well as stemness- and invasiveness-related genes.

Another aspect of this invention refers to the use of a compound of formula (I) as defined above, or a pharmaceutically acceptable salt, stereoisomer or solvate thereof, in the manufacture of a medicament, particularly a medicament for the treatment and/or prophylaxis of a disease associated with deregulation of p73 as well as stemness- and invasiveness-related genes.

Another aspect of the present invention refers to a method for the treatment and/or prophylaxis of a disease associated with deregulation of p73 as well as stemness- and invasiveness-related genes, the method comprising administering to the subject in need of such a treatment or prophylaxis an effective amount of a compound of formula (I) as defined above, or a pharmaceutically acceptable salt, stereoisomer or solvate thereof.

The present invention also relates to the obtention of compounds of formula (I) from microorganisms capable of producing them and to the obtention of compounds of formula (I) by synthesis/derivatization from the natural compounds obtained from microorganisms, specifically from a fungus identified as *Chaetomium sp.*, and more specifically from the strain of *Chaetomium sp.* HT-GU-SUES-904. The process for obtaining a compound of formula (I), or a pharmaceutically acceptable salt, stereoisomer or solvate thereof, comprises the steps of:
i) cultivating the fungal strain *Chaetomium sp.* HT-GU-SUES-904, available under accession number CECT 20840 from the Colección Española de Cultivos Tipo at Valencia University, Spain, in an aqueous nutrient medium, to obtain a natural compound of formula (I) in a culture broth and
ii) recovering the resulting natural compound of formula (I) from the culture broth;
   or
   i) cultivating the fungal strain *Chaetomium sp.* HT-GU-SUES-904, available under accession number CECT 20840 from the Colección Española de Cultivos Tipo at Valencia University, Spain, in an aqueous nutrient medium, to obtain a natural compound in a culture broth and
   ii) either recovering the resulting natural compound from the culture broth and modifying it to obtain a compound of formula (I) or
      modifying the resulting natural compound to obtain a compound of formula (I) in a reaction crude and recovering the compound of formula (I) from the reaction crude.

Another aspect of the invention is the strain of *Chaetomium sp.* HT-GU-SUES-904, available under accession number CECT 20840 from the Colección Española de Cultivos Tipo at Valencia University, Spain.

These and other aspects of the invention and preferred embodiments thereof are additionally also defined hereinafter in the detailed description and in the claims.

All the features described in this specification (including the claims, description and drawings) can be combined in any combination, with the exception of combinations of such mutually exclusive features.

### BRIEF DESCRIPTION OF DRAWINGS

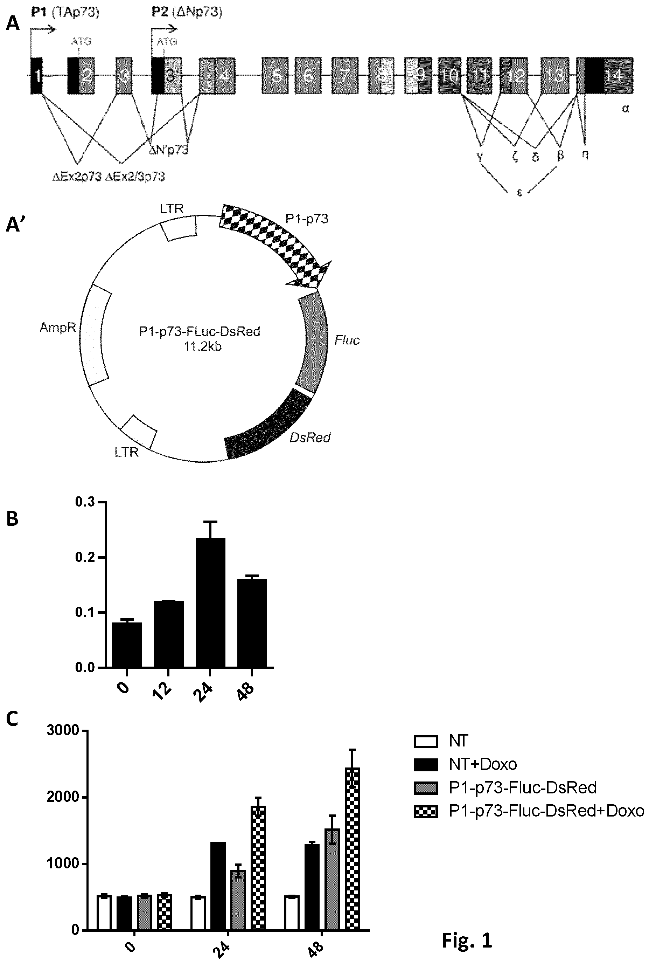

**Figure 1** **illustrates the structure of the P1-p73-Fluc-DsRed reporter vector and the validation to monitor the isoforms generated from the P1 promoter of *TP73* gene: TAp73, ΔEx2p73, ΔEx2/3p73, and ΔN'p73.** (A) Scheme of the human *TP73* gene structure (from Horvat et al., 2021) depicting the p73 isoforms transcribed from the P1 promoter upstream of exon 1: the full length TAp73, and the N-terminally truncated isoforms ΔEx2p73, ΔEx2/3p73, and ΔN'p73 generated by alternative splicing. (A') Map of the lentiviral reporter vector P1-p73-Fluc-DsRed with the P1 promoter (P1) of *TP73* driving the expression of firefly luciferase (*Fluc*) and the red fluorescent protein DsRed. (B) Monitorization of the P1-promoter activity in HCT116 p53-/- cells by measuring endogenous TAp73 expression normalized by the 18S ribosomal RNA (Y axis) after doxorubicin (Doxo) treatment (know activator of TAp73 expression at RNA level) by qRT-PCR analysis at the indicated time points (X axis, hours). (C) Monitorization of the P1-promoter activity using the reporter vector in HCT116 p53-/- cells. Fluorescence levels (DsRed, Y axis) were measured by fluorimetry in non-transfected cells (NT) and in cells transfected with the P1-p73-Fluc-DsRed reporter, after Doxo treatment at the indicated time points (X axis). Values are expressed as mean ± standard error of the mean (SEM).

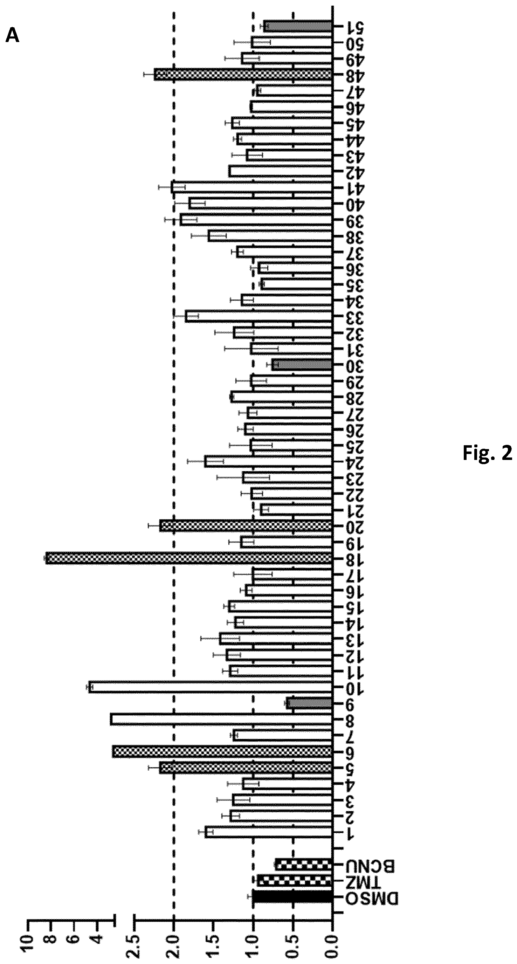

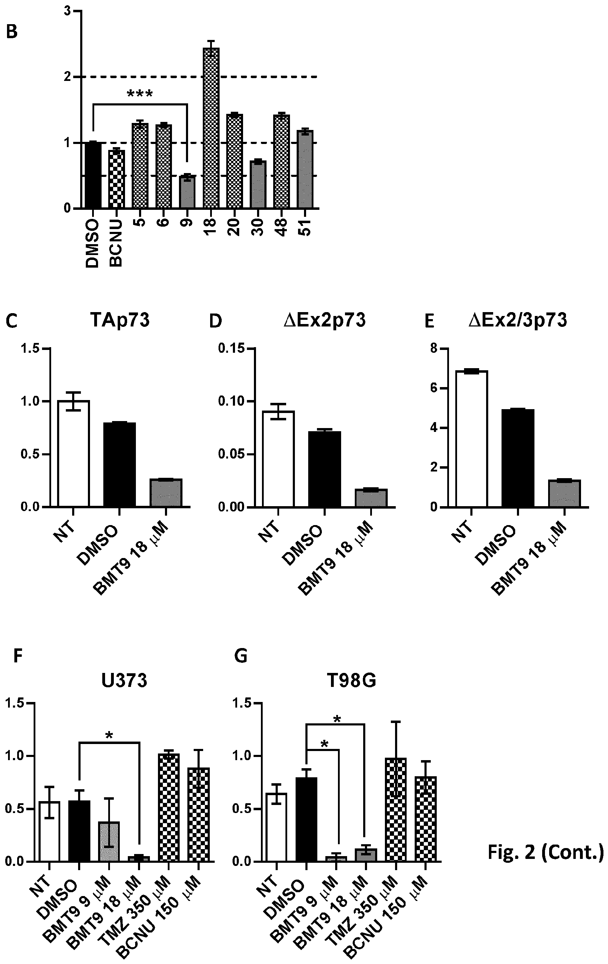

**Figure 2** **shows the results of the screening of 51 diverse natural compounds from Instituto Biomar S.A. (BMT 1 to 51) in the U373 human glioblastoma cell line to identify compounds able to modulate P1-*TP73* promoter activity**. (A) Cells were transfected with the reporter vector and treated either with the compounds (BMT 1-51), the first line drugs against glioblastoma (temozolomide-TMZ or carmustine-BCNU) or the vehicle control (Dimetil sulfoxide, DMSO). Fluorimetry was measured 48 h after treatment. (A-B) Relative fluorescence (DsRed, Y axis) in transfected cells after treatment (X axis, treatment). Fluorescence levels were normalized to those of DMSO treated cells. (B) The compounds with a modulatory effect of a 2-fold fluorescence induction (square fill pattern bars) or a 0.5-fold reduction (grey bars) were pre-selected, and the assay was repeated as in (A). Drugs with autofluorescence were discarded (BMT-8, -10). (C-E) Effect of BMT9 treatment on the p73 isoform's level generated from the P1-promoter: TAp73 (C), ΔEx2p73 (D) and ΔEx2/3p73 (E), in U373 cells under the same treatment conditions as in (A) and (B). mRNA expression levels (Y axis) were measured by qRT-PCR, using 18S ribosomal RNA as housekeeping control. The represented values are relative to TAp73 expression levels in the non-treated cells (NT) in (C). The most prominently expressed isoforms were TAp73 and ΔEx2/3p73, but treatment reduced them all. ΔN'p73 was barely detected. (F-G) Quantification of endogenous TAp73 protein levels in U373 (F) and T98G (G) glioblastoma cells analyzed by Western blot after 72 h of the indicated treatments (X axis). TAp73 expression was normalized to GAPDH and the ratio of band intensities is represented (Y axis). Values are expressed as mean ± standard error of the mean (SEM). Differences were considered significant when p < 0.05 (*p < 0.05). **BMT9, but not the first line drugs TMZ or BCNU, reduced the expression of P1-driven p73 isoforms; in particular, the compound efficiently reduced TAp73 expression in conventional glioblastoma cell lines.**

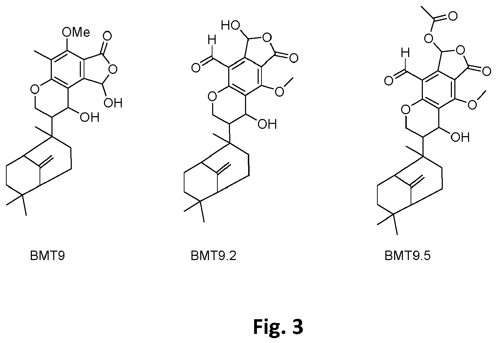

**Figure 3** **depicts the chemical structure of the BMT9 and two analogues [BMT9.2 and BMT9.5], encompassed within general formula (I) according to the present invention.**

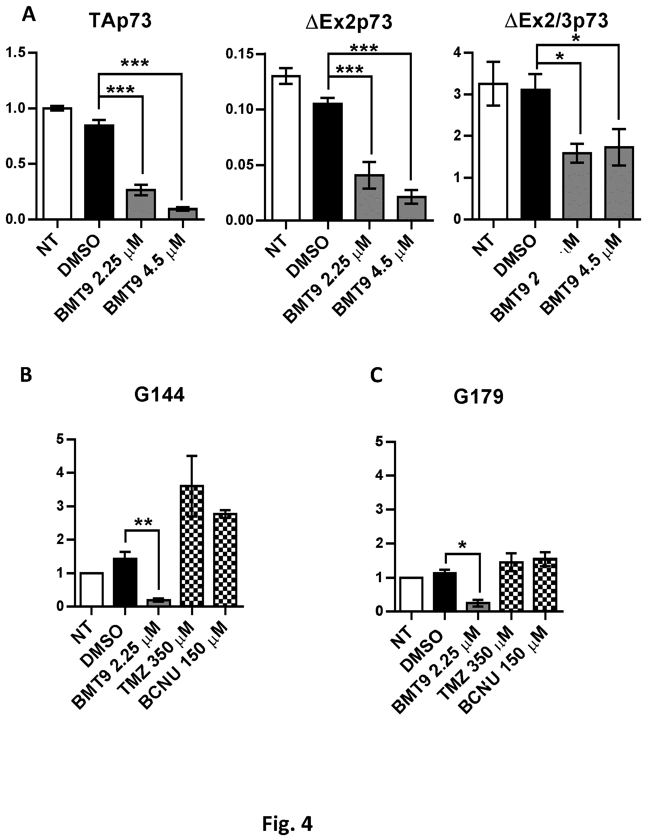

**Figure 4** **demonstrates that BMT9 significantly reduces P1-promoter driven isoforms, TAp73 in particular, in glioblastoma neural stem cell lines G144 and G179.** (A) Effect of BMT9 treatment (X axis) on the expression of the indicated p73 isoforms generated from the P1-*TP73* promoter in G144 cells. Expression levels were analyzed by qRT-PCR and normalized to the 18S ribosomal RNA. Relative values to TAp73 expression in non-treated cells (NT) in (A) are represented (Y axis). As previously observed for the conventional glioblastoma lines, the expression of all isoforms was reduced by BMT9 treatment, with TAp73 and ΔEx2/3p73 being the predominantly affected isoforms, while ΔN'p73 was barely detected (not shown). (B-C) TAp73 protein levels after treatment (X axis) with either BMT9, TMZ (350 µM) or BCNU (150 µM) in G144 (B) or G179 (C) cells. mRNA fold-decrease relative to TAp73 expression values in the non-treated cells (NT) was calculated (Y axis), using GAPDH as a loading control. Values are expressed as mean ± standard error of the mean (SEM). Differences were considered significant when p < 0.05 (*p < 0.05, **p < 0.01, ***p< 0.001). **These results revealed BMT9 as a promising candidate compound to reduce the expression of P1-driven p73 isoforms, in particular, TAp73, in glioblastoma neural stem cells.**

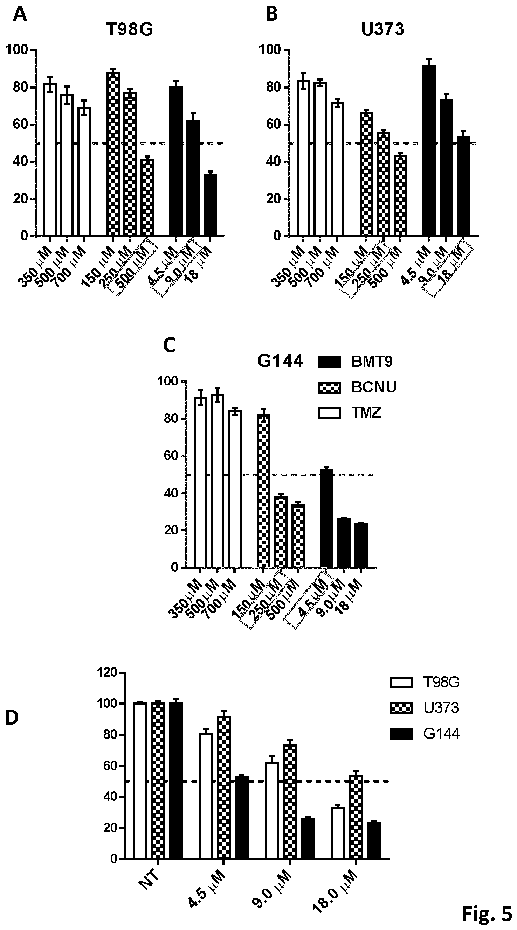

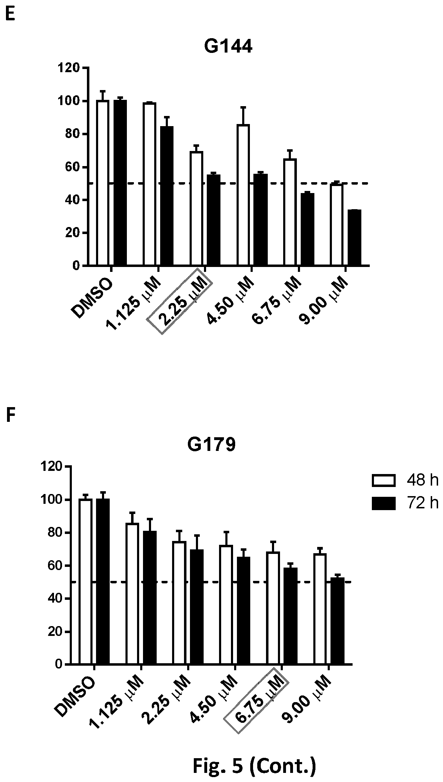

**Figure 5** **demonstrates that BMT9 is more effective against glioblastoma neural stem cells (GNSC) than first line drugs Temozolomide (TMZ) and Carmustine (BCNU).** (A-C) Comparative dose-dependent effect of BMT9 (black bars), BCNU (square fill pattern bars) or TMZ (white bars) in conventional cell lines T98G (A), U373 (B), or the Glioblastoma neural stem cell line G144 (C). Cells were treated at the indicated concentrations of compounds (X axis) and cell viability was measured by MTT assay 72 h later (Y axis, percentage of cell viability relative to non-treated cells). DMSO treatment had no effect on cell viability. The IC50, when reached, is marked by a grey rectangle. BMT9 IC50 is lower than BCNU IC50 in T98G (A, 55-times reduction), U373 (B, 14-times reduction) and G144 (C, 55-times reduction), respectively. (D) Representation of BMT9 effect on cell viability in the three cell lines (data from A-C), indicating that BMT9 is more effective against GNSC than against conventional glioblastoma cell lines. Nevertheless, at 18 µM it reaches the IC50 in all of them. (E-F) Lower concentrations of BMT9 (X axis) still reduced the viability of GNSC, G144 and G179, after 72 h of treatment (Y axis, percentage of cell viability relative to DMSO-treated cells). Values are expressed as mean ± standard error of the mean (SEM).

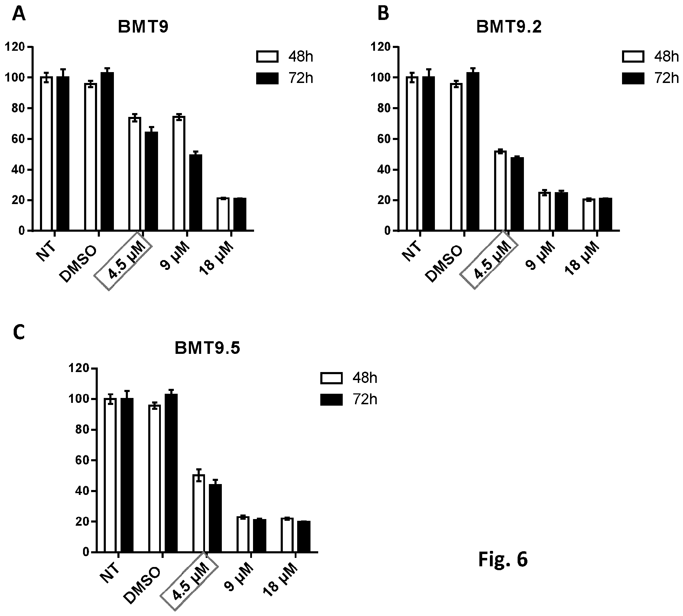

**Figure 6** **shows that BMT9 analogues BMT9.2 and BMT9.5 are also effective against GNSC.** (A-C) Comparison of the effectiveness of BMT9 treatment (A) with its analogues: BMT9.2 (B) and BMT9.5 (C). Cell viability was measured by MTT assay 48 and 72 h after treatment (Y axis, percentage of cell viability relative to non-treated cells-NT). Values are expressed as mean ± standard error of the mean (SEM).

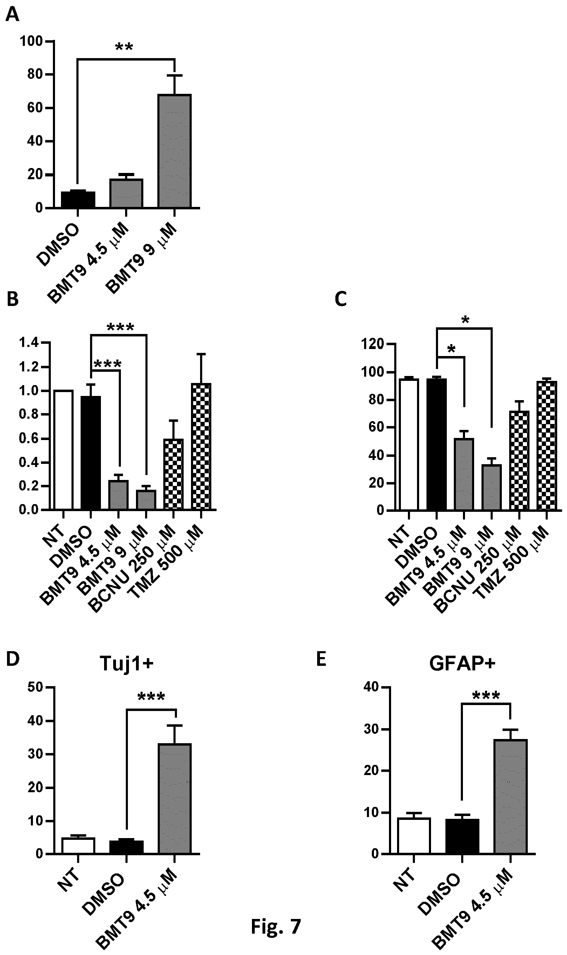

**Figure 7** **reveals that BMT9 treatment (IC50) moderately induces apoptosis and reduces the stemness-related marker CD133 and induces neural differentiation in GNSC.** (A) Percentage of apoptotic cells (Propidium iodide/Anexin V double positive population, Y axis) measured by flow cytometry after DMSO or BMT9 treatment for 48 h at the indicated concentrations (X axis). (B-C) Treatment of G144 cells with BMT9, but not with TMZ or BCNU, reduces the expression levels of the stemness marker CD133 (B), as well as the % of CD133 positive cells (C). Flow cytometry analysis was used to quantify the Mean Fluorescence Intensity (B, Y axis, values relative to the non-treated control, NT) and the percentage of CD133-positive cells (C, Y axis) after 48 h of the indicated treatments (X axis). (D-E) BMT9 treatment (4.5 µM) induces the expression of neural (Tuj1, D) and astrocytic (GFAP, E) markers in G144 cells. Protein expression was analyzed by immunostaining and confocal microscopy 72h after treatment and positive cells were quantified manually. At least a total of 300 cells were quantified. Vertical axis represents the percentage of Tuj1 or GFAP positive cells for each condition (X axis). Analysis was performed using a Zeiss LSM800 confocal microscope. Values are expressed as mean ± standard error of the mean (SEM). Differences were considered significant when p < 0.05 (**p-value < 0.01; ***p-value < 0.001).

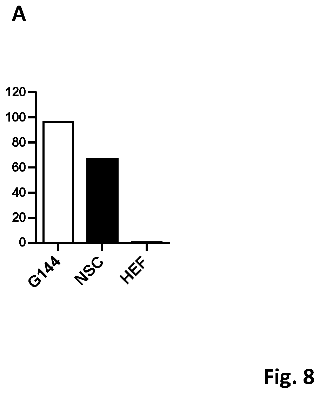

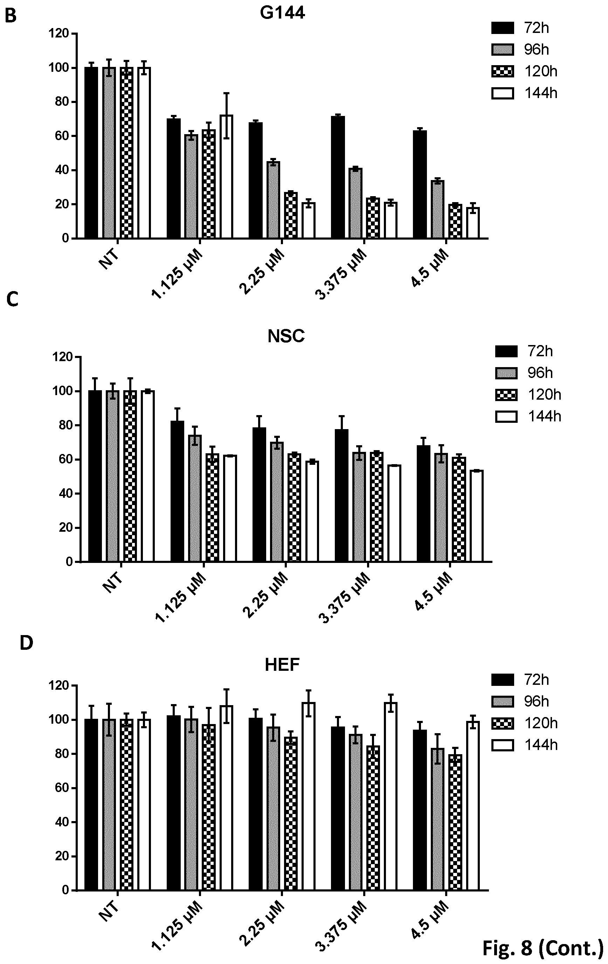

**Figure 8** **indicates that low concentrations of BMT9 efficiently affects CD133-positive GNSC in long term cultures.** (A) Flow cytometry analysis of the percentage of CD133 positive population (Y axis) in the studied cell lines: G144 Glioblastoma neural stem cells (GNSC), healthy Neural Stem Cells (NSC) and Human Embryonic Fibroblast (HEF). (B-D) Comparative dose-dependent effect on cellular viability (Y axis, MTT values relative to non-treated cells, NT) after BMT9 treatment (1.25-4.5 µM, X axis) at the indicated time points of long-term culture. Values are expressed as mean ± standard error of the mean (SEM). **BMT9 affected CD133-positive NSC and GNSC, but with a stronger effect against GNSC, indicating that there is a therapeutic window of treatment.**

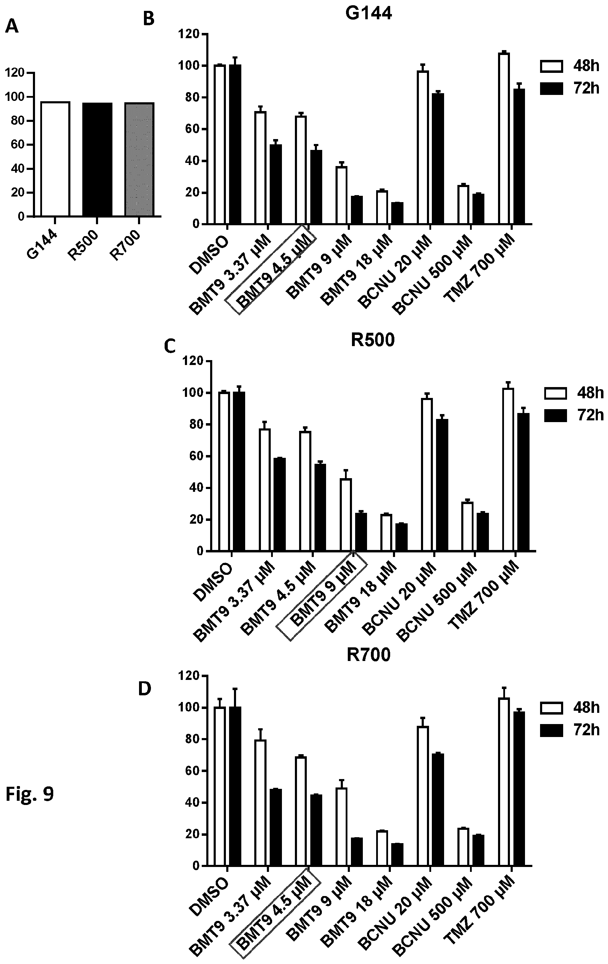

**Figure 9** **shows that CD133⁺-G144-TZM resistant clones remain sensitive to BMT9.** (A) TMZ resistant clones, (G144-TMZ-R500 and G144-TMZ-R700) were generated after the serial treatment of G144 parental cells with 500 µM and 700 µM concentrations of TMZ, respectively. The percentage of CD133 positive population was quantified by flow cytometry in the G144 parental cells and the resistant cell lines. (B-D) Comparative effects on cellular viability by MTT assay (Y axis, relative MTT values to DMSO treated cells) of a range of BMT9, TMZ and BCNU concentrations (X axis) on G144 parental (B), G144-TMZ-R500 (C) and G144-TMZ-R700 (D) cells at 48 and 72 hours after treatment. For comparative purposes, in addition to the known BCNU IC50 (500 µM), a lower concentration of BCNU was used (20 µM BCNU is in close range to concentrations of BMT9 used).

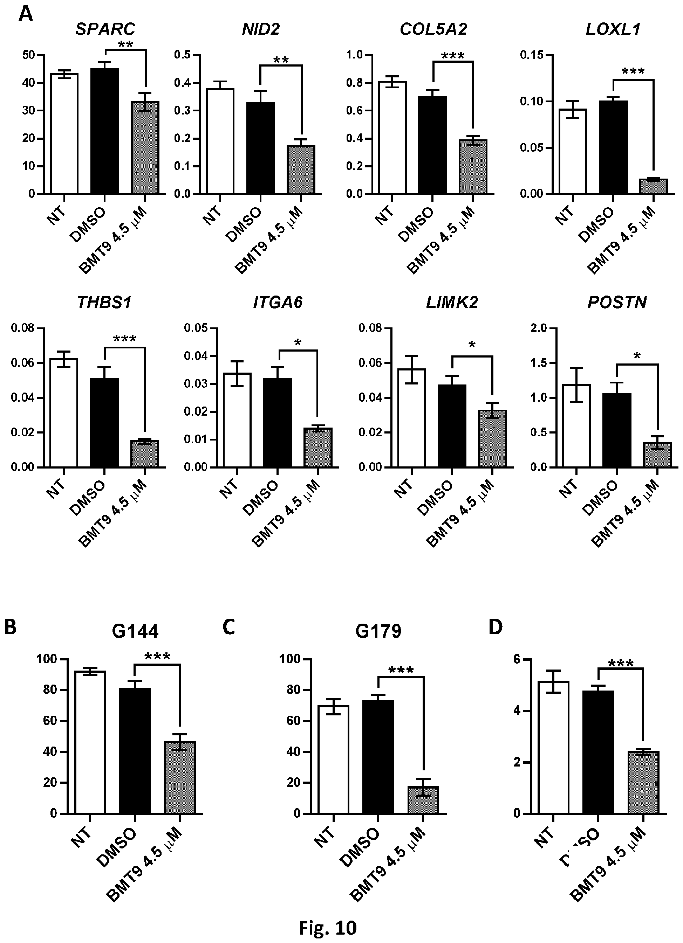

**Figure 10** **reveals that BMT9 reduces the expression of invasiveness related genes in GNSC and impairs cell migration and invasiveness capacity *in vitro.*** (A) Expression analysis of the indicated invasiveness associated genes in G144 cells after BMT9 treatment for 72 h (Y axis, mRNA expression levels quantified by qRT-PCR and normalized by 18S ribosomal RNA values). (B-D) BMT9 reduces GNSC migration (B-C) and invasiveness capacity (D). Wound healing assay in G144 (B) and G179 (C) cell lines determined as the percentage of wound closure (Y axis) after 24 hours in the presence of the indicated treatment (X axis; NT: non-treated cells). (D) Cell invasion assay of G144 cells cultured in three-dimensional spheroid cultures embedded in Matrigel and treated as indicated (X axis). The initial area of each sphere and the invaded area after 24 h-treatment were quantified using Image J software analysis. The ratio between both areas is represented (Y axis). Values are expressed as mean ± standard error of the mean (SEM). Differences were considered significant when p < 0.05 (*p-value < 0.05; **p-value < 0.01; ***p-value < 0.001).

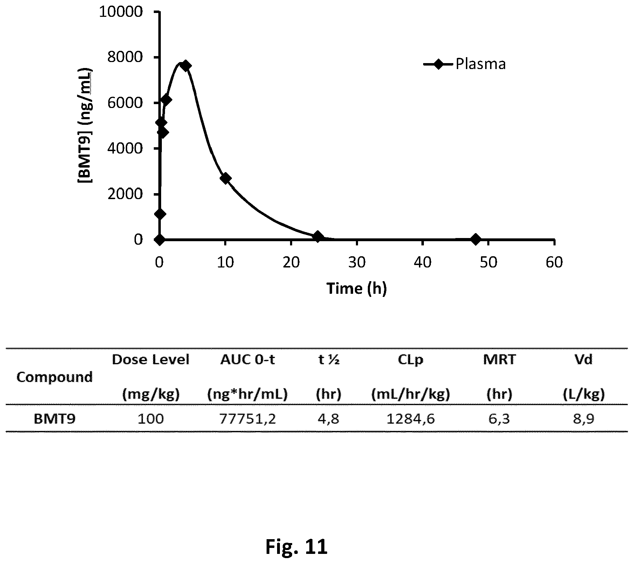

**Figure 11** **represents the concentration of BMT9 in plasma of female C57BL/6 mice collected at t = 0 min, 5 min, 15 min, 30 min, 1 hour, 4 hours, 10 hours, 24 hours and 48 hours.** Pharmacokinetics (PK) profile and main PK parameters are shown. The fundamental pharmacokinetic parameters, half-life (t1/2), clearance (CLp), MRT (mean residence time) volume of distribution (Vd) and AUC (area under the curve) were obtained from the non-compartmental analysis using PK Solutions 2.0.2.

### DETAILED DESCRIPTION OF THE INVENTION

Cancer is a leading cause of human death. The inventors propose that downregulation of TAp73 and other isoforms generated by the *TP73* gene promoter could be an appropriate pathway to target for therapeutic intervention in cancer and related diseases. Additionally, the control of cell stemness and invasion could be further enhanced by downregulation of stemness- and invasiveness-related genes.

The present invention relates to novel compounds which are defined by the general formula (I) and are able to treat and/or prevent diseases associated with deregulation of p73, as well as stemness- and invasiveness-related genes, such as cancer.

In these compounds the groups can be selected in accordance with the following guidance:
The term "hydroxyl protecting group" (HPG) refers to a group blocking the OH function that can be removed under controlled conditions. Suitable protecting groups are well known in the art. A general review of protecting groups in organic chemistry is provided by Wuts, PGM and Greene TW in Greene's Protecting Groups in Organic Synthesis, 4th Ed. 2006 Wiley-Interscience, and by Kocienski PJ in Protecting Groups, 3rd Ed. 2005 Georg Thieme Verlag. These references provide sections on protecting groups for hydroxy groups. Virtually any hydroxyl protecting group can be used to put the invention into practice. Examples of such protected OH include ethers, silyl ethers, esters, carbonates, carbamates, sulfonates, sulfinates and sulfenates,

Illustrative, non-limiting examples of HPGs include:
- ethers [-R'], including alkoxy and aryloxy methyl ethers [-CH₂-OR']. R' can be selected from C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl and 3- to 10-membered heteroaryl. Examples of ethers include methyl ether, tert-butyl ether, benzyl ether, p-methoxybenzyl ether, 3,4-dimethoxybenzyl ether, trityl ether, allyl ether, methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers;
- silyl ethers [-Si(R')(R")(R‴)]. R', R" and R‴ can be independently selected from C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl and halogen. Examples of silyl ethers include trimethylsilyl ether, triethylsilyl ether, tert-butyldimethylsilyl ether, tert-butyldiphenylsilyl ether, tri-isopropylsilyl ether, diethylisopropylsilyl ether, hexyldimethylsilyl ether, triphenylsilyl ether, di-tert-butylmethylsilyl ether;
- esters [-COR']. R' can be selected from C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl and 3- to 10-membered heteroaryl. Examples of esters include acetate ester, benzoate ester, pivalate ester, methoxyacetate ester, chloroacetate ester, levulinate ester;
- carbonates [-COOR']. R' can be selected from C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl and 3- to 10-membered heteroaryl. Examples of carbonates include benzyl carbonate, p-nitrobenzyl carbonate, tert-butyl carbonate, 2,2,2-trichloroethyl carbonate, 2-(trimethylsilyl)ethyl carbonate, allyl carbonate;
- carbamates [-CON(R')(R")]. R' and R" can be independently selected from H, C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl and 3- to 10-membered heteroaryl; and
- sulfonates, sulfinates and sulfenates [-S(O)ₙR', with n = 2, 1 or 0, respectively]. R' can be selected from H, C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl and 3- to 10-membered heteroaryl.

As the skilled person knows, hydroxyl protecting groups are commonly named considering the oxygen atom. Thus, terms such as "ether", "silyl ether", "ester", "carbonate", "carbamate", "sulfonate", "sulfinate" and "sulfenate" really refer herein to the chemical group formed with the oxygen atom i.e., -OR, -OSi(R)(R')(R"), -OC(=O)R, - OC(=O)OR, -OCON(R)(R'), -OS(O)₂R, -OS(O)R, or -OSR, respectively.

In the case of ethers the protecting group for the OH can be selected for instance from methyl, methoxymethyl, methylthiomethyl, (phenyldimethylsilyl)methoxymethyl, benzyloxymethyl, *p*-methoxybenzyloxymethyl, [(3,4-dimethoxybenzyl)oxy]methyl, *p-*nitrobenzyloxymethyl, o-nitrobenzyloxymethyl, [(*R*)-1-(2-nitrophenyl)ethoxy]methyl, (4-methoxyphenoxy)methyl, guaiacolmethyl, [(*p*-phenylphenyl)oxy]methyl, *t*-butoxymethyl, 4-pentenyloxymethyl, siloxymethyl, 2-methoxyethoxymethyl, 2-cyanoethoxymethyl, bis(2-chloroethoxy)methyl, 2,2,2-trichloroethoxymethyl, 2-(trimethylsilyl)ethoxymethyl, menthoxymethyl, *o*-bis(2-acetoxyethoxy) methyl, tetrahydropyranyl, fluorous tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl S,S-dioxide, 1-[(2-chloro-4-methyl)phenyl]-4-methoxypiperidin-4-yl, 1-(2-fluorophenyl)-4-methoxypiperidin-4-yl, 1-(4-chlorophenyl)-4-methoxypiperidin-4-yl, 1,4-dioxan-2-yl, tetrahydrofuranyl, tetrahydrothiofuranyl, 2,3,3a,4,5,6,7,7*a*-octahydro-7,8,8-trimethyl-4,7-methanobenzofuran-2-yl, 1-ethoxyethyl, 1-(2-chloroethoxy)ethyl, 2-hydroxyethyl, 2-bromoethyl, 1-[2-(trimethylsilyl)ethoxy]ethyl, 1-methyl-1-methoxyethyl, 1-methyl-1-benzyloxyethyl, 1-methyl-1-benzyloxy-2-fluoroethyl, 1-methyl-1-phenoxyethyl, 2,2,2-trichloroethyl, 1,1-dianisyl-2,2,2-trichloroethyl, 1,1,1,3,3,3-hexafluoro-2-phenylisopropyl, 1-(2-cyanoethoxy)ethyl, 2-trimethylsilylethyl, 2-(benzylthio)ethyl, 2-phenylselenyl)ethyl, *t*-butyl, cyclohexyl, 1-methyl-1'-cyclopropylmethyl, allyl, prenyl, cinnamyl, 2-phenallyl, propargyl, *p-*chlorophenyl, *p*-methoxyphenyl, *p*-nitrophenyl, 2,4-dinitrophenyl, 2,3,5,6-tetrafluoro-4-(trifluoromethyl)phenyl, benzyl, *p*-methoxybenzyl, 3,4-dimethoxybenzyl, 2,6-dimethoxybenzyl, *o*-nitrobenzyl, *p*-nitrobenzyl, pentadienylnitrobenzyl, pentadienylnitropiperonyl, halobenzyl, 2,6-dichlorobenzyl, 2,4-dichlorobenzyl, 2,6-difluorobenzyl, *p*-cyanobenzyl, fluorous benzyl, 4-fluorousalkoxybenzyl, trimethylsilylxylyl, *p*-phenylbenzyl, 2-phenyl-2-propyl, *p*-acylaminobenzyl, *p*-azidobenzyl, 4-azido-3-chlorobenzyl, 2-trifluoromethylbenzyl, 4-trifluoromethylbenzyl, *p-*(methylsulfinyl)benzyl, *p*-siletanylbenzyl, 4-acetoxybenzyl, 4-(2-trimethylsilyl)ethoxymethoxybenzyl, 2-naphthylmethyl, 2-picolyl, 4-picolyl, 3-methyl-2-picolyl *N*-oxido, 2-quinolinylmethyl, 6-methoxy-2-(4-methylphenyl-4-quinolinemethyl, 1-pyrenylmethyl, diphenylmethyl, 4-methoxydiphenylmethyl, 4-phenyldiphenylmethyl, *p*,*p*'-dinitrobenzhydryl, 5-dibenzosuberyl, triphenylmethyl, tris(4-*t*-butylphenyl)methyl, α-naphthyldiphenylmethyl, *p*-methoxyphenyldiphenylmethyl, di(*p-*methoxyphenyl)phenylmethyl, tri(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 4,4'-dimethoxy-3"-[*N*-(imidazolylmethyl)]trityl, 4,4'-dimethoxy-3"-[*N*-(imidazolylethyl)carbamoyl]trityl, bis(4-methoxyphenyl)-1'-pyrenylmethyl, 4-(17-tetrabenzo[*a*,*c*,*g*,*i*]fluorenylmethyl)-4,4"-dimethoxytrityl, 9-anthryl, 9-(9-phenyl)xanthenyl, 9-phenylthioxanthyl, 9-(9-phenyl-10-oxo)anthryl, 1,3-benzodithiolan-2-yl, and 4,5-bis(ethoxycarbonyl)-[1,3]-dioxolan-2-yl, benzisothiazolyl *S*,*S*-dioxido. In the case of silyl ethers the protecting group for the OH can be selected for instance from trimethylsilyl, triethylsilyl, triisopropylsilyl, dimethylisopropylsilyl, diethylisopropylsilyl, dimethylhexylsylil, 2-norbornyldimethylsilyl, *t*-butyldimethylsilyl, *t-*butyldiphenylsilyl, tribenzylsilyl, tri-*p*-xylylsilyl, triphenylsilyl, diphenylmethylsilyl, di-*t-*butylmethylsilyl, bis(*t*-butyl)-1-pyrenylmethoxysilyl, tris(trimethylsilyl)silyl, (2-hydroxystyryl)dimethylsilyl, (2-hydroxystyryl)diisopropylsilyl, *t*-butylmethoxyphenylsilyl, *t-*butoxydiphenylsilyl, 1,1,3,3-tetraisopropyl-3-[2-(triphenylmethoxy)ethoxy]disiloxane-1-yl, and fluorous silyl. In the case of esters the protecting group for the OH can be selected for instance from formate, benzoylformate, acetate, chloroacetate, dichloroacetate, trichloroacetate, trichloroacetamidate, trifluoroacetate, methoxyacetate, triphenylmethoxyacetate, phenoxyacetate, *p*-chlorophenoxyacetate, phenylacetate, diphenylacetate, 3-phenylpropionate, bisfluorous chain type propanoyl, 4-pentenoate, 4-oxopentanoate, 4,4-(ethylenedithio)pentanoate, 5[3-bis(4-methoxyphenyl)hydroxymethylphenoxy]levulinate, pivaloate, 1-adamantoate, crotonate, 4-methoxycrotonate, benzoate, *p*-phenylbenzoate, 2,4,6-trimethylbenzoate, 4-bromobenzoate, 2,5-difluorobenzoate, *p*-nitrobenzoate, picolinate, nicotinate, 2-(azidomethyl)benzoate, 4-azidobutyrate, (2-azidomethyl)phenylacetate, 2-{[(tritylthio)oxy]methyl}benzoate, 2-{[(4-methoxytritylthio)oxy]methyl}benzoate, 2-{[methyl(tritylthio)amino]methyl}benzoate, 2-{{[(4-methoxytrityl)thio]methylamino}-methyl}benzoate, 2-(allyloxy)phenylacetate, 2-(prenyloxymethyl)benzoate, 6-(levulinyloxymethyl)-3-methoxy-2-nitrobenzoate, 6-(levulinyloxymethyl)-3-methoxy-4-nitrobenzoate, 4-benzyloxybutyrate, 4-trialkylsilyloxybutyrate, 4-acetoxy-2,2-dimethylbutyrate, 2,2-dimethyl-4-pentenoate, 2-iodobenzoate, 4-nitro-4-methylpentanoate, *o*-(dibromomethyl)benzoate, 2-formylbenzenesulfonate, 4-(methylthiomethoxy)butyrate, 2-(methylthiomethoxymethyl)benzoate, 2-(chloroacetoxymethyl)benzoate, 2-[(2-chloroacetoxy)ethyl]benzoate, 2-[2-(benzyloxy)ethyl]benzoate, 2-[2-(4-methoxybenzyloxy)ethyl]benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (*E*)-2-methyl-2-butenoate, *o*-(methoxycarbonyl)benzoate, α-naphthoate, nitrate, alkyl *N*,*N*,*N*,*N*-tetramethylphosphorodiamidate, and 2-chlorobenzoate. In the case of carbonates the protecting group for the OH can be selected for instance from methyl carbonate, methoxymethyl carbonate, 9-fluorenylmethyl carbonate, ethyl carbonate, bromoethyl carbonate, 2-(methylthiomethoxy)ethyl carbonate, 2,2,2-trichloroethyl carbonate, 1,1-dimethyl-2,2,2-trichloroethyl carbonate, 2-(trimethylsilyl)ethyl carbonate, 2-[dimethyl(2-naphthylmethyl)silyl]ethyl carbonate, 2-(phenylsulfonyl) ethyl carbonate, 2-(triphenylphosphonio)ethyl carbonate, *cis-*[4-[[(methoxytrityl)sulfenyl]oxy]tetrahydrofuran-3-yl]oxy carbonate, isobutyl carbonate, *t-*butyl carbonate, vinyl carbonate, allyl carbonate, cinnamyl carbonate, propargyl carbonate, *p*-chlorophenyl carbonate, *p*-nitrophenyl carbonate, 4-ethoxy-1-naphthyl carbonate, 6-bromo-7-hydroxycoumarin-4-ylmethyl carbonate, benzyl carbonate, *o-*nitrobenzyl carbonate, *p*-nitrobenzyl carbonate, *p*-methoxybenzyl carbonate, 3,4-dimethoxybenzyl carbonate, anthraquinon-2-ylmethyl carbonate, 2-dansylethyl carbonate, 2-(4-nitrophenyl)ethyl carbonate, 2-(2,4-dinitrophenyl)ethyl carbonate, 2-(2-nitrophenyl)propyl carbonate, alkyl 2-(3,4-methylenedioxy-6-nitrophenyl)propyl carbonate, 2-cyano-1-phenylethyl carbonate, 2-(2-pyridyl)amino-1-phenylethyl carbonate, 2-[*N*-methyl-*N*-(2-pyridyl)]amino-1-phenylethyl carbonate, phenacyl carbonate, 3',5'-dimethoxybenzoin carbonate, methyl dithiocarbonate, and *S*-benzyl thiocarbonate. In the case of carbamates the protecting group for the OH can be selected for instance from dimethylthiocarbamate, *N*-phenylcarbamate, *N*-methyl-*N*-(*o-*nitrophenyl)carbamate. In the case of sulfonates, sulfenates and sulfinates the protecting group for the OH can be selected for instance from sulfate, allylsulfonate, methanesulfonate, benzylsulfonate, tosylate, 2-[(4-nitrophenyl)ethyl]sulfonate, 2-trifluoromethylbenzenesulfonate, 4-monomethoxytritylsulfenate, alkyl 2,4-dinitrophenylsulfenate, 2,2,5,5-tetramethylpyrrolidin-3-one-1-sulfinate, and dimethylphosphinothiolyl. The mention of these groups should be not interpreted as a limitation of the scope of the invention, since they have been mentioned as a mere illustration of protecting groups for OH, but further groups having said function may be known by the skilled person in the art, and they are to be understood to be also encompassed by the present invention.

Likewise, by way of non-limiting example, hydroxyl protecting groups include methyl, methoxylmethyl (MOM), methylthiomethyl (MTM), *t*-butylthiomethyl, (phenyldimethylsilyl)methoxymethyl (SMOM), benzyloxymethyl (BOM), *p-*methoxybenzyloxymethyl (PMBM), (4-methoxyphenoxy)methyl (*p*-AOM), guaiacolmethyl (GUM), *t*-butoxymethyl, 4-pentenyloxymethyl (POM), siloxymethyl, 2-methoxyethoxymethyl (MEM), 2,2,2-trichloroethoxymethyl, bis(2-chloroethoxy) methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, benzyl, *p*-methoxybenzyl, 3,4-dimethoxybenzyl, *o*-nitrobenzyl, *p*-nitrobenzyl, *p-*halobenzyl, 2,6-dichlorobenzyl, *p*-cyanobenzyl, *p*-phenylbenzyl, 2-picolyl, 4-picolyl, 3-methyl-2-picolyl N-oxido, diphenylmethyl, *p,p*'-dinitrobenzhydryl, 5-dibenzosuberyl, triphenylmethyl, α-naphthyldiphenylmethyl, *p*-methoxyphenyldiphenylmethyl, di(*p-*methoxyphenyl)phenylmethyl, tri(p-methoxyphenyl)methyl, 4-(4'-bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4- methoxyphenyl)-1'-pyrenylmethyl, 9-anthryl, 9-(9-phenyl)xanthenyl, 9-(9-phenyl-10- oxo)anthryl, 1,3-benzodithiolan-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, *t*-butyldimethylsilyl (TBDMS), *t-*butyldiphenylsilyl (TBDPS), tribenzylsilyl, tri-p-xylylsilyl, triphenylsilyl, diphenylmethylsilyl (DPMS), t-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4- methoxycrotonate, benzoate, *p*-phenylbenzoate, 2,4,6-trimethylbenzoate (mesitoate), alkyl methyl carbonate, 9-fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl)ethyl carbonate (Psec), 2-(triphenylphosphonio) ethyl carbonate (Peoc), alkyl isobutyl carbonate, alkyl vinyl carbonate alkyl allyl carbonate, alkyl *p*-nitrophenyl carbonate, alkyl benzyl carbonate, alkyl *p*-methoxybenzyl carbonate, alkyl 3,4-dimethoxybenzyl carbonate, alkyl *o*-nitrobenzyl carbonate, alkyl *p*-nitrobenzyl carbonate, alkyl S-benzyl thiocarbonate, 4-ethoxy-1-napththyl carbonate, methyl dithiocarbonate, 2-iodobenzoate, 4-azidobutyrate, 4-nitro-4-methylpentanoate, *o-*(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate, o-(methoxycarbonyl)benzoate, α-naphthoate, nitrate, alkyl *N,N,N',N'*-tetramethylphosphorodiamidate, alkyl *N-*phenylcarbamate, borate, dimethylphosphinothioyl, alkyl 2,4-dinitrophenylsulfenate, sulfate, methanesulfonate (mesylate), benzylsulfonate, and tosylate (Ts). For protecting 1,2- or 1,3-diols, the protecting groups include methylene acetal, ethylidene acetal, 1-t-butylethylidene ketal, 1-phenylethylidene ketal, (4- methoxyphenyl)ethylidene acetal, 2,2,2-trichloroethylidene acetal, acetonide, cyclopentylidene ketal, cyclohexylidene ketal, cycloheptylidene ketal, benzylidene acetal, *p*-methoxybenzylidene acetal, 2,4-dimethoxybenzylidene ketal, 3,4-dimethoxybenzylidene acetal, 2-nitrobenzylidene acetal, methoxymethylene acetal, ethoxymethylene acetal, dimethoxymethylene ortho ester, 1-methoxyethylidene ortho ester, 1-ethoxyethylidine ortho ester, 1,2-dimethoxyethylidene ortho ester, α-methoxybenzyhdene ortho ester, 1-(*N,N-*dimethylamino)ethylidene derivative, α-(*N,N*'-dimethylamino)benzylidene derivative, 2-oxacyclopentylidene ortho ester, di-*t*-butylsilylene group (DTBS), 1,3-(1,1,3,3-tetraisopropyldisiloxanylidene) derivative (TIPDS), tetra-t-butoxydisiloxane-1,3-diylidene derivative (TBDS), cyclic carbonates, cyclic boronates, ethyl boronate, and phenyl boronate.

The term "alkyl" refers to a linear or branched alkane derivative containing from 1 to 6 ("C₁-C₆ alkyl"), preferably from 1 to 3 ("C₁-C₃ alkyl"), carbon atoms and which is bound to the rest of the molecule through a single bond. Illustrative examples of alkyl groups include methyl, ethyl, n-propyl, i-propyl, n-butyl, i-butyl, t-butyl, pentyl, hexyl.

The term "alkenyl" refers to a linear or branched hydrocarbon chain radical containing from 2 to 6 ("C₂-C₆ alkenyl"), preferably from 2 to 3 ("C₂-C₃ alkenyl"), carbon atoms and which contains at least one double bond and is attached to the rest of the molecule by a single bond. Examples of alkenyl groups include ethenyl, propenyl, allyl, butenyl, 1-methyl-2-buten-1-yl, and the like.

The term "cycloalkyl" refers to a radical derived from cycloalkane containing from 3 to 7 ("C₃-C₇ cycloalkyl"), preferably from 3 to 6 ("C₃-C₆ cycloalkyl") carbon atoms. Illustrative examples of cycloalkyl groups include cyclopropyl, cyclobutyl, cyclopentyl, cyclohexyl, etc.

The term "aryl" refers to an aromatic group having between 6 and 10, preferably 6 or 10 carbon atoms, comprising 1 or 2 aromatic nuclei, bound by means of a carbon-carbon bond or fused. Illustrative examples of aryl groups include phenyl, naphthyl, diphenyl, indenyl, etc. Preferably, it is phenyl.

The term "arylalkyl" refers to an alkyl group as defined above substituted with an aryl group as defined above, such as e.g. (C₆-C₁₀)aryl(C₁-C₆)alkyl and (C₆-C₁₀)aryl(C₁-C₃)alkyl. Examples of such groups include benzyl, phenylethyl, phenylpropyl, naphthylmethyl, etc. Preferably, it is benzyl.

The term "heterocyclyl" refers to a saturated or partially unsaturated monocyclic or bicyclic system containing from 3 to 10, preferably 5 to 7, ring atoms containing one or more, specifically one, two, three or four ring heteroatoms independently selected from N, O, and S, and the remaining ring atoms being carbon. Illustrative examples of heterocyclyl groups include tetrahydropyran, morpholine, piperazine, piperidine and [1,4]dioxane.

The term "heteroaryl" refers to an aromatic monocyclic or bicyclic system containing from 3 to 10, preferably 5 to 7, ring atoms containing one or more, specifically one, two, three or four ring heteroatoms independently selected from O, N and S, and the remaining ring atoms being carbon. Illustrative examples of heteroaryl groups include pyrrole, furan, thiophene, pyridine and pyrimidine.

The term "halogen" refers to bromine, chlorine, iodine or fluorine.

As understood in this technical area, there may be a certain degree of substitution in the aforementioned radicals. Therefore, there may be substitution in any of the groups of the present invention. The previous groups can be substituted in one or more available positions with one or more substituents. Said substituents include, for example and in non-limiting sense, C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl, halogen, OR, =O, SR, SOR, SO₂R, OSO₂R, OSO₃R, NO₂, NHR, N(R)₂, =N-R, N(R)COR, N(COR)₂, N(R)SO₂R, N(R)C(=NR)N(R)R, N₃, CF₃, CN, COR, COOR, OCOR, OCOOR, OCONHR, OCON(R)₂, CONHR, CON(R)₂, CON(R)OR, CON(R)SO₂R, PO(OR)₂, PO(OR)R, PO(OR)(N(R)R), wherein each of the R groups is independently selected from hydrogen, C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl and trifluoromethyl.

By "pharmaceutically acceptable" is meant herein a material that is not biologically or otherwise undesirable, i. e., the material may be incorporated into a pharmaceutical composition administered to a patient without causing any undesirable biological effects or interacting in a deleterious manner with any of the other components of the composition in which it is contained. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The invention also provides "salts" of the compounds described in the present description. By way of illustration, said salts can be acid addition salts, base addition salts or metal salts, and can be synthesized from the parent compounds containing a basic or acid moiety by means of conventional chemical processes known in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, *p*-toluenesulfonate, trifluoroacetate, camphorsulfonate, etc. Illustrative examples of base addition salts include inorganic base salts such as, for example, ammonium salts and organic base salts such as, for example, ethylenediamine, ethanolamine, *N*,*N*-dialkylenethanolamine, triethanolamine, glutamine, amino acid basic salts, etc. Illustrative examples of metal salts include, for example, sodium, potassium, calcium, magnesium, aluminum and lithium salts.

The term "solvate" according to this invention is to be understood as meaning any form of the compound which has another molecule (most likely a polar solvent) attached to it via non-covalent bonding. Examples of solvate include hydrates and alcoholates, e.g. methanolates. Methods of solvation are generally known within the art. The compounds of the invention may present different polymorphic forms, and it is intended that the invention encompasses all such forms.

The term "stereoisomer" as used herein includes any enantiomer, diastereomer or geometric isomer (E/Z) of such compound. In particular, compounds referred to herein may have asymmetric centres and therefore exist in different enantiomeric or diastereomeric forms. Thus, any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same or different than the stereoisomerism of the other double bonds of the molecule. All the stereoisomers including enantiomers, diastereoisomers and geometric isomers of the compounds referred to herein, and mixtures thereof, are considered within the scope of the present invention.

In a particular embodiment, the compound of the invention is selected from a compound of general formula (Ia): 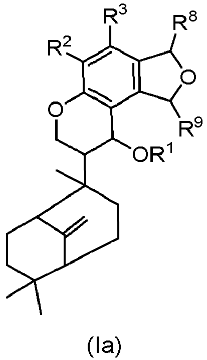 wherein
R¹ is hydrogen or a hydroxyl protecting group:
R² and R³ are independently selected from the group consisting of hydrogen, C₁-C₆ alkyl, -CHO, -COOH and -OR⁶, wherein R⁶ is hydrogen or a hydroxyl protecting group; and
R⁸ and R⁹ are independently selected from the group consisting of hydrogen, =O or -OR⁷, wherein R⁷ is hydrogen or a hydroxyl protecting group;
or a pharmaceutically acceptable salt, stereoisomer or solvate thereof.

In a particular embodiment, the compound of the invention is selected from a compound of general formula (Ib): 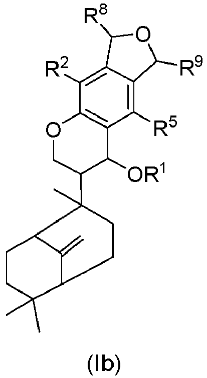 wherein
R¹ is hydrogen or a hydroxyl protecting group:
R² and R⁵ are independently selected from the group consisting of hydrogen, C₁-C₆ alkyl, -CHO, -COOH and -OR⁶, wherein R⁶ is hydrogen or a hydroxyl protecting group; and
R⁸ and R⁹ are independently selected from the group consisting of hydrogen, =O or -OR⁷, wherein R⁷ is hydrogen or a hydroxyl protecting group;
or a pharmaceutically acceptable salt, stereoisomer or solvate thereof.

In a particular embodiment, the compound of the invention is selected from a compound of general formula (Ic): 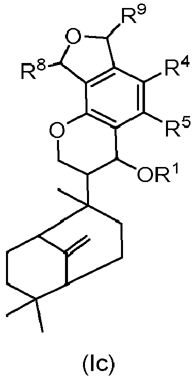 wherein
R¹ is hydrogen or a hydroxyl protecting group:
R⁴ and R⁵ are independently selected from the group consisting of hydrogen, C₁-C₆ alkyl, -CHO, -COOH and -OR⁶, wherein R⁶ is hydrogen or a hydroxyl protecting group; and
R⁸ and R⁹ are independently selected from the group consisting of hydrogen, =O or -OR⁷, wherein R⁷ is hydrogen or a hydroxyl protecting group
or a pharmaceutically acceptable salt, stereoisomer or solvate thereof.

In the compounds of the invention, preferably each hydroxyl protecting group is independently selected from the group consisting of ethers, silyl ethers, esters, carbonates, carbamates, sulfonates, sulfinates and sulfonates, as previously defined.

Illustrative, non-limiting examples of HPGs include:
- ethers [-R'], including alkoxy and aryloxy methyl ethers [-CH₂-OR']. R' can be selected from C₁-C₆ alkyl, C₆-C₁₀ aryl and (C₆-C₁₀)aryl(C₁-C₆)alkyl;
- silyl ethers [-Si(R')(R")(R‴)]. R', R" and R'" can be independently selected from C₁-C₆ alkyl, C₆-C₁₀ aryl and (C₆-C₁₀)aryl(C₁-C₆)alkyl;
- esters [-COR']. R' can be selected from C₁-C₆ alkyl, C₆-C₁₀ aryl and (C₆-C₁₀)aryl(C₁-C₆)alkyl;
- carbonates [-COOR']. R' can be selected from C₁-C₆ alkyl, C₆-C₁₀ aryl and (C₆-C₁₀)aryl(C₁-C₆)alkyl;
- carbamates [-CON(R')(R")]. R' and R" can be independently selected from H, C₁-C₆ alkyl, C₆-C₁₀ aryl and (C₆-C₁₀)aryl(C₁-C₆)alkyl; and
- sulfonates, sulfinates and sulfenates [-S(O)ₙR', with n = 2, 1 or 0, respectively]. R' can be selected from H, C₁-C₆ alkyl, C₆-C₁₀ aryl and (C₆-C₁₀)aryl(C₁-C₆)alkyl.

Particular examples of preferred HPGs are C₁-C₆ alkyl (e.g. methyl) ethers and C₁-C₆ alkyl (e.g methyl) esters.

In a particular embodiment, the compound of the invention meets at least one of the following conditions:
- R¹ is hydrogen;
- at least one of R², R³, R⁴, and R⁵ is -OR⁶, wherein R⁶ is hydrogen or C₁-C₆ alkyl, preferably methyl;
- at least one of R², R³, R⁴, and R⁵ is C₁-C₆ alkyl, preferably methyl or -CH₂OH;
- at least one of R², R³, R⁴, and R⁵ is -CHO;
- at least one of R², R³, R⁴, and R⁵ is -COOR, wherein R is selected from H, C₁-C₆ alkyl, C₆-C₁₀ aryl and (C₆-C₁₀)aryl(C₁-C₆)alkyl;
- at least one of R⁸ and R⁹ is =O; and/or
- at least one of R⁸ and R⁹ is -OR⁷, wherein R⁷ is selected from hydrogen, C₁-C₆ alkyl, preferably methyl, and -COR', wherein R' is selected from C₁-C₆ alkyl, preferably methyl, C₆-C₁₀ aryl and (C₆-C₁₀)aryl(C₁-C₆)alkyl.

In a particular embodiment, R¹ is hydrogen in the compounds of the invention.

In a particular embodiment of the compounds of formula (la),
R² is C₁-C₆ alkyl, preferably methyl, and/or
R³ is -OR⁶, wherein R⁶ is hydrogen or C₁-C₆ alkyl, preferably methyl.

In a particular embodiment of the compounds of formula (la),
R⁸ is =O and/or
R⁹ is -OR⁷, wherein R⁷ is hydrogen or C₁-C₆ alkyl, preferably methyl.

In a particular embodiment of the compounds of formula (la),
R² is C₁-C₆ alkyl, preferably methyl, and/or
R³ is -OR⁶, wherein R⁶ is hydrogen or C₁-C₆ alkyl, preferably methyl, and/or
R⁸ is =O and/or
R⁹ is -OR⁷, wherein R⁷ is hydrogen or C₁-C₆ alkyl, preferably methyl.

In a particular embodiment of the compounds of formula (lb),
R² is -CHO, and/or
R⁵ is -OR⁶, wherein R⁶ is hydrogen or C₁-C₆ alkyl, preferably methyl.

In a particular embodiment of the compounds of formula (lb),
R⁸ is -OR⁷, wherein R⁷ is selected from hydrogen and C₁-C₆ alkyl, preferably methyl, and/or
R⁹ is =O.

In a particular embodiment of the compounds of formula (lb),
R² is -CHO, and/or
R⁵ is -OR⁶, wherein R⁶ is hydrogen or C₁-C₆ alkyl, preferably methyl, and/or
R⁸ is -OR⁷, wherein R⁷ is selected from hydrogen and C₁-C₆ alkyl, preferably methyl, and/or
R⁹ is =O.

In a particular embodiment of the compounds of formula (lb),
R² is C₁-C₆ alkyl, preferably -CH₂OH, and/or
R⁵ is -OR⁶, wherein R⁶ is hydrogen or C₁-C₆ alkyl, preferably methyl.

In a particular embodiment of the compounds of formula (lb),
R⁸ is -OR⁷, wherein R⁷ is selected from hydrogen and C₁-C₆ alkyl, preferably methyl, and/or
R⁹ is =O.

In a particular embodiment of the compounds of formula (lb),
R² is C₁-C₆ alkyl, preferably -CH₂OH, and/or
R⁵is -OR⁶, wherein R⁶ is hydrogen or C₁-C₆ alkyl, preferably methyl, and/or
R⁸ is -OR⁷, wherein R⁷ is selected from hydrogen and C₁-C₆ alkyl, preferably methyl, and/or
R⁹ is =O.

In a particular embodiment of the compounds of formula (lb),
R² is -COOH, and/or
R⁵ is -OR⁶, wherein R⁶ is hydrogen or C₁-C₆ alkyl, preferably methyl.

In a particular embodiment of the compounds of formula (lb),
R⁸ is -OR⁷, wherein R⁷ is selected from hydrogen and C₁-C₆ alkyl, preferably methyl, and/or
R⁹ is hydrogen.

In a particular embodiment of the compounds of formula (lb),
R² is -COOH, and/or
R⁵ is -OR⁶, wherein R⁶ is hydrogen or C₁-C₆ alkyl, preferably methyl, and/or
R⁸ is -OR⁷, wherein R⁷ is selected from hydrogen and C₁-C₆ alkyl, preferably methyl, and/or
R⁹ is hydrogen.

In a particular embodiment of the compounds of formula (lb),
R⁸ is -OR⁷, wherein R⁷-COR wherein R is C₁-C₆ alkyl, preferably methyl, and/or
R⁹ is =O.

In a particular embodiment of the compounds of formula (lb),
R² is -CHO, and/or
R⁵ is -OR⁶, wherein R⁶ is hydrogen or C₁-C₆ alkyl, preferably methyl, and/or
R⁸ is -OR⁷, wherein R⁷-COR wherein R is C₁-C₆ alkyl, preferably methyl, and/or
R⁹ is =O.

Particular individual compounds of the invention falling under formula (I) include the following: 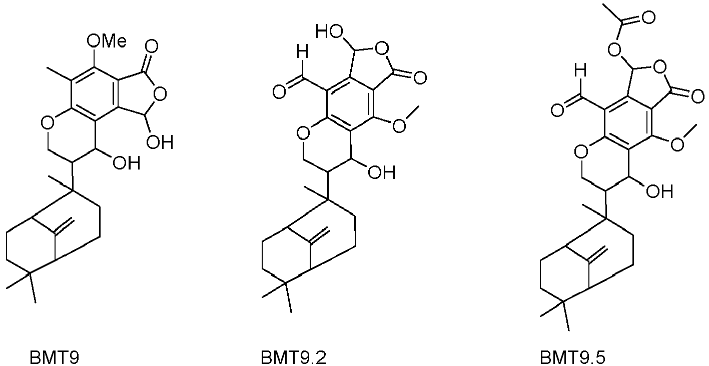 or a pharmaceutically acceptable salt, stereoisomer or solvate thereof.

Specifically, BMT9 is encompassed within general formula (Ia) whereas BMT9.2 and BMT9.5 are encompassed within general formula (Ib).

An important feature of the above described compounds is their bioactivity and in particular their ability to modulate and suppress the transcriptional activity of P1-TP73 gene promoter, reducing the expression of P1-driven p73 isoforms, as well as its activity modulating the expression of stemness- and invasiveness-related signature genes, and therefore their utility in therapy against diseases associated with deregulation of p73 as well as stemness- and invasiveness-related genes, such as cancer.

Thus, a further aspect of the present invention relates to a medicament or composition in different pharmaceutical forms comprising at least a compound of general formula (I) or a pharmaceutically acceptable salt, stereoisomer or solvate thereof, optionally at least another drug and at least one pharmaceutically acceptable excipient.

Examples of pharmaceutical compositions include any solid (tablets, pills, capsules, granules, etc.), semi-solid (creams, ointments, etc.) or liquid (solutions, suspensions or emulsions) composition.

The compositions can for example be administered orally, topically, dermally, nasally, intravenously, intramuscularly, intraperitoneally, intracerobrospinally, intracranially, intraspinally, subcutaneously, intraarticularly, intrasynovialy, or intrathecaly. Other forms of administration are not excluded.

The respective composition or medicament may - depending on its route of administration - also contain one or more excipients known to those skilled in the art. The composition or medicament according to the present invention may be produced according to standard procedures known to those skilled in the art.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, ^{©} 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

This invention also refers to a compound of general formula (I), or a pharmaceutically acceptable salt, stereoisomer or solvate thereof, for use in medicine, particularly for the treatment and/or prophylaxis of diseases associated with deregulation of p73 as well as stemness- and invasiveness-related genes.

Likewise, this invention also refers to the use of a compound of general formula (I), or a pharmaceutically acceptable salt, stereoisomer or solvate thereof, in the manufacture of a medicament, particularly a medicament for the treatment and/or prophylaxis of a disease associated with deregulation of p73 as well as stemness- and invasiveness-related genes.

As used herein, the terms "treat", "treating" and "treatment" include in general the eradication, removal, reversion, alleviation, modification, or control of a disease, particularly a disease associated with deregulation of p73 as well as stemness- and invasiveness-related genes, after its onset.

As used herein, the terms "prevention", "preventing", "preventive", "prevent" and "prophylaxis" refer to the capacity of a given substance, composition or medicament to avoid, minimize or difficult the onset or development of a disease, particularly a disease associated with deregulation of p73 as well as stemness- and invasiveness-related genes, before its onset.

Diseases associated with deregulation of p73 as well as stemness- and invasiveness-related genes include cancer and related diseases. For instance, p73 has been identified as a player in Niemann-Pick type C disease, palmoplantar pustulosis, autoimmune encephalitis and other disorders.

The term "cancer", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. Cancers usually share some of the following characteristics: sustaining proliferative signaling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumor cells resemble, which is therefore presumed to be the origin of the tumor.

Examples of cancer or tumor include without limitation, brain, breast, heart, lung, small intestine, colon, stomach, esophageal, spleen, kidney, bladder, head, neck, ovarian, prostate, rectum, pancreas, skin, bone, bone marrow, blood, thymus, cervical, uterus, testicles, hepatobiliary and liver tumors.

In particular, the tumor/cancer can be selected from the group of cervical cancer (Wei et al., 2012), leukemia (e.g. B-cell chronic lymphocytic leukemia), ovarian carcinoma, gastric adenocarcinoma, medulloblastoma, glioblastoma, glioma, astrocytoma, ependymoma, oligodendroglioma, breast invasive carcinoma, stomach adenocarcinoma, lung squamous cell carcinoma, colon adenocarcinoma, esophageal carcinoma tumors, adenoma, angiosarcoma, epithelial carcinoma, germinoma, hemangioendothelioma, hepatoblastoma, lymphoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytictumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelial tumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, undifferentiated carcinoma, uveal melanoma, verrucous carcinoma, vipoma, Wilm's tumor.

More particularly, the tumor/cancer can be selected from the group of cervical cancer (Wei et al., 2012), B-cell chronic lymphocytic leukemia, ovarian carcinomas, gastric adenocarcinoma, medulloblastoma, glioblastoma, glioma, astrocytoma, ependymoma, and oligodendroglioma breast invasive carcinoma, stomach adenocarcinoma, lung squamous cell carcinoma, colon adenocarcinoma, and esophageal carcinoma tumors.

In a particular embodiment, the brain cancer is selected from glioblastoma, glioma, astrocytoma, ependymoma, and oligodendroglioma, more particularly, glioblastoma.

In an embodiment, the compound of formula (I) or a pharmaceutically acceptable salt, stereoisomer or solvate thereof, or its pharmaceutical composition or medicament, is intended to be used as as suppressor of the transcriptional activity of P1-*TP73* gene promoter, and therefore to be applied as inhibitors of all the isoforms generated by this promotor such as, but not limited to, TAp73, ΔEx2p73, ΔEx2/3p73, y ΔN'p73.

In an embodiment, the compound of formula (I) or a pharmaceutically acceptable salt, stereoisomer or solvate thereof, or its pharmaceutical composition or medicament, is intended to be used in a cancer/tumor with glutamine addiction in which p73 overexpression results in support of cancer cell survival upon metabolic stress.

In an embodiment, the compound of formula (I) or a pharmaceutically acceptable salt, stereoisomer or solvate thereof, or its pharmaceutical composition or medicament, is intended to be used in diseases in which p73 overexpression causes metabolic alterations as in cancer.

In an embodiment, the compound of formula (I) or a pharmaceutically acceptable salt, stereoisomer or solvate thereof, or its pharmaceutical composition or medicament, is intended to the be used in the treatment of a disease with alteration of TAp73-dependent regulation of pro-inflammatory cytokines and immunomodulatory molecules, such as palmoplantar pustulosis or autoimmune encephalitis.

In an embodiment, the compound of formula (I) or a pharmaceutically acceptable salt, stereoisomer or solvate thereof, or its pharmaceutical composition or medicament, is intended to be used in the treatment of a cancer/tumor in which Cancer Stem Cells play a role in its dissemination and propagation such as, but not limited to glioblastoma, head and neck cancer, colon cancer.

In an embodiment, the compound of formula (I) or a pharmaceutically acceptable salt, stereoisomer or solvate thereof, or its pharmaceutical composition or medicament, is intended to be used in a cancer/tumor comprising presence of Cancer Stem Cells, preferably CD133-CSC or wherein the cancer/tumor has its origin in CD133-positive neural stem cells (NSC) or neural progenitor cells (NPC).

In an embodiment, the compound of formula (I) or a pharmaceutically acceptable salt, stereoisomer or solvate thereof, or its pharmaceutical composition or medicament, is intended to reduce the expression of invasiveness related genes such as, but not limited to, *SPARC, NID2, COL5A2, LOXL1, THBS1, ITGA6, LIMK2, POSTN.*

In an embodiment, the compound of formula (I) or a pharmaceutically acceptable salt, stereoisomer or solvate thereof, or its pharmaceutical composition or medicament, is intended to reduce the expression of stemness related genes such as, but not limited to, *ABAT, ABCG2, AGT, ARHGAP11A, ASCL1, ASPM, ATAD2, ATP1B2, AURKA, BCAN, BIRC5, BRCA1, BUB1, BUB1B, CALM1, CCNA2, CCNB1, CCNB2, CDC20, CDC7, CDCA2, CDKN3, CENPA, CENPF, CENPH, CENPK, CEP55, CKAP2L, CKS2, DCX, DDX3X, DHFR, DIAPH3, DLGAP5, DLL1, DLL3, DTL, ECT2, ELM01, EPAS1, ESPL1, FANCI, FBXO5, FEN1, FOXM1, FXYD6, GGH, GINS2, GMNN, GPSM2, GRIA2, HAS2, HES6, HMMR, IDH1, IGFBPL1, KIF11, KIF14, KIF15, KIF18A, KIF20A, KIF23, KIF2C, KIF4A, KNTC1, LIG1, LGR5, LRRN1, MAD2L1, MAGED1, MARCKS, MCAM, MCM2, MCM3, MCM4, MCM6, MELK, MEST, METTL7B, MND1, MLLT11, MSH2, NCAPG, NCAPH, NDC80, NEK2, NES, NEU4, NFIA, NMU, NUF2, NXPH1, OLIG1, PAICS, PBK, PCNA, PDSS1, PIK3R3, PMP2, POLQ, PRIM1, PROM1, PTPRZ1, PTTG1, PUS7, RACGAP1, RAD51, RAD51AP1, RBMX, RCC1, RRM2, SEMA6D, SEZ6L, SHD, SOX1, SOX4, SOX9, SKP2, SMC2, SMC4, TIMELESS, TM4SF1, TMEM97, TNR, TOP2A, TPX2, TRIP13, TROAP, TTK, TUBA1A, TUBB2A, TYMS, UBE2C, WDR34, ZWINT,* and more particularly, *ASCL1, BUB1, CDC20, KIF14, KIF18A, LGR5, PROM1, SOX1, SOX4, SOX9, OLIG1, CDKN3, CKAP2L, DLGAP5, TTK.*

In an embodiment, the compound of formula (I) or a pharmaceutically acceptable salt, stereoisomer or solvate thereof, or its pharmaceutical composition or medicament, is intended to reduce the stemness and/or invasiveness of Cancer Stem Cells.

Stemness generally refers to the molecular processes underlying the fundamental stem cell (SC) properties of self-renewal, the ability of a cell to perpetuate its lineage and pluripotency to give rise to differentiated cells. In Cancer Stem Cells (CSCs) stemness properties sustain cancer progression, such as enhanced capacities for self-renewal cloning, growing, metastasizing, homing, and reproliferating (Aponte & Caicedo, 2017).

Invasiveness generally refers to the capacity of cancer cells to spread beyond the tissue where the cancer first developed to healthy surrounding tissues

In an embodiment, the compound of formula (I) or a pharmaceutically acceptable salt, stereoisomer or solvate thereof, or its pharmaceutical composition or medicament, is intended to be used in a tumor/cancer which is resistant to current chemotherapeutic treatments (such as first line drugs Temozolomide (TMZ) and Carmustine (BCNU)).

In an embodiment, the compound of formula (I) or a pharmaceutically acceptable salt, stereoisomer or solvate thereof, or its pharmaceutical composition or medicament, is intended to be used in a tumor/cancer in which the tumor status of O6-methylguanine-methyltransferase (MGMT) influences the efficacy of treatment with alkylating agents such as, but not limited to, carmustine and temozolomide.

Also disclosed is a method for the treatment of a patient, notably a human, suffering a disease associated with deregulation of p73 as well as stemness- and invasiveness-related genes or likely to suffer a disease associated with deregulation of p73 as well as stemness- and invasiveness-related genes, which comprises administering to the patient in need of such a treatment or prophylaxis an effective amount of a compound of general formula (I) or a pharmaceutically acceptable salt, stereoisomer or solvate thereof.

By an "effective" amount or a "therapeutically effective amount" of a drug or pharmacologically active agent is meant a nontoxic but sufficient amount of the drug or agent to provide the desired effect. In the therapy of the present invention, an "effective amount" of the compound of formula (I) is the amount of that compound that is effective to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

The present invention also encompasses the combination of the compounds of formula (I) with other drugs (e.g. an anticancer drug). A combination of at least a compound of formula (I) and at least another drug may be formulated for its simultaneous, separate or sequential administration. This has the implication that the combination of the two compounds may be administered:
- as a combination that is being part of the same medicament formulation, the two compounds being then administered always simultaneously.
- as a combination of two units, each with one of the substances giving rise to the possibility of simultaneous, sequential or separate administration.

In a particular embodiment, the compound of formula (I) is independently administered from the other drug (i.e in two units) but at the same time.

In another particular embodiment, the compound of formula (I) is administered first, and then the other drug is separately or sequentially administered.

In yet another particular embodiment, the other drug is administered first, and then the compound of formula (I) is administered, separately or sequentially, as defined.

Compounds of formula (I) can be produced using microorganisms and also can be produced by synthesis/derivatization from the natural compounds obtained from microorganisms. Preferably, the organism used for the production of compounds of formula (I) is the fungal strain *Chaetomium sp.* HT-GU-SUES-904, available under accession number CECT 20840 from the Colección Española de Cultivos Tipo at Valencia University, Spain.

In particular, certain compounds of the invention such as BMT9 or BTM9.2 may be produced by cultivating the strain *Chaetomium sp.* HT-GU-SUES-904, in a suitable nutrient medium, such as those described in the examples, until a significant amount accumulates in the fermentation.

Then, the natural compound obtained is recovered from the culture broth and optionally it is isolated/separated and purified.

Isolation/separation and purification of compounds can be performed using conventional techniques such as chromatography, precipitation or crystallization, preferably by chromatography.

Additionally, compounds of the invention can be obtained by modifying those already obtained from the natural source by using standard organic synthetic reactions known by the skilled person, for instance by means of derivatization, protection/deprotection and isomerisation reactions. Thus, hydroxyl groups can be acylated by standard coupling or acylation procedures, for instance by using acetic acid, acetyl chloride or acetic anhydride in pyridine or the like. Formate groups can be obtained by heating hydroxyl precursors in formic acid. Carbamates can be obtained by heating hydroxyl precursors with isocyanates. Hydroxyl groups can be converted into halogen groups through intermediate sulfonates for iodide, bromide or chloride, or directly using a sulfur trifluoride for fluorides; or they can be reduced to hydrogen by reduction of intermediate sulfonates. Hydroxyl groups can also be converted into alkoxy groups by alkylation using an alkyl bromide, iodide or sulfonate, or into amino lower alkoxy groups by using, for instance, a protected 2-bromoethylamine. Amide groups can be alkylated or acylated by standard alkylation or acylation procedures, for instance by using, respectively, KH and methyl iodide or acetyl chloride in pyridine or the like. Ester groups can be hydrolized to carboxylic acids or reduced to aldehyde or to alcohol. Carboxylic acids can be coupled with amines to provide amides by standard coupling or acylation procedures. Carbonyl compounds can be reduced to alcohols by standard procedures. Double bonds can be epoxidized by known methods. When necessary, appropriate protecting groups can be used on the substituents to ensure that reactive groups are not affected. The procedures and reagents needed to prepare these derivatives are known to the skilled person and can be found in general textbooks such as March's Advanced Organic Chemistry 6th Edition 2007, Wiley Interscience. As the skilled person will appreciate, certain compounds of formula (I) may be useful as intermediate products in the preparation of other compounds of formula (I).

In a particular embodiment, the process for obtaining compound of formula (I), or a pharmaceutically acceptable salt, stereoisomer or solvate thereof, comprises:
i) cultivating the fungal strain *Chaetomium sp.* HT-GU-SUES-904, available under accession number CECT 20840 from the Colección Española de Cultivos Tipo at Valencia University, Spain, in an aqueous nutrient medium, with assimilable carbon and nitrogen sources and salts under aerobic and mesophilic conditions, preferably between 24 and 28 °C at a pH 3.5 to 8.8, to obtain a natural compound of formula (I) in a culture broth;
ii) recovering the resulting natural compound of formula (I) from the culture broth; and
iii) optionally, isolating and purifying the compound of formula (I);
   or
   i) cultivating the fungal strain *Chaetomium sp.* HT-GU-SUES-904, available under accession number CECT 20840 from the Colección Española de Cultivos Tipo at Valencia University, Spain, in an aqueous nutrient medium with assimilable carbon and nitrogen sources and salts under aerobic and mesophilic conditions, preferably between 24 and 28 °C at a pH 3.5 to 8.8, to obtain a natural compound in a culture broth and
   ii) either recovering the resulting natural compound from the culture broth and modifying it to obtain a compound of formula (I) or
      modifying the resulting natural compound to obtain a compound of formula (I) in a reaction crude and recovering the compound of formula (I) from the reaction crude; and
   iii) optionally, isolating and purifying the compound of formula (I).

Other compounds of this invention can be prepared using techniques already known in the art. As examples, said compounds can be made by modifications of the conditions of the fermentation or by conventional chemical methods using compounds of formula (I) as starting materials, or by synthesis, from commercially available products and reagents, using standard organic synthesis reactions known by the skilled person such as those described in "Comprehensive Organic Transformations: A Guide to Functional Group Preparations", 2nd Edition, Richard C. Larock, or in "March's Advanced Organic Chemistry: Reactions, Mechanisms, and Structure", 6th Edition , Michael B. Smith, and Jerry March, or in "Advanced Organic Chemistry, Part B: Reactions and Synthesis", 5th Edition, Francis A. Carey, and Richard J. Sundberg.

One aspect of the present invention is said isolated strain of *Chaetomium sp.* HT-GU-SUES-904, deposited at the Colección Española de Cultivos Tipo with accession number CECT 20840 as well as a culture thereof including a biologically pure culture. A description of this microorganism is provided hereinafter in the experimental part.

The term "biologically pure culture", as used herein, refers to a culture in which the fungus strain of the invention is found in a ratio of 90% or over, for example 95 % or over, 96% or over, 97% or over, 98% or over, 99% or over or 100%, compared to other organisms present in the culture.

While the deposited strain is clearly preferred, the preparation of the compounds of the present invention is not restricted or limited to any particular strain or organisms. It is the intention of the present invention to include preparation processes using other producing organisms, strains or mutants.

The following examples are merely illustrative of certain embodiments of the invention and cannot be considered as restricting it in any way.

### EXAMPLES

### Example 1: Description and Fermentation of HT-GU-SUES-904

This invention describes new compounds isolated from the fermentation broth of a microorganism, a fungal strain *Chaetomium sp.* HT-GU-SUES-904, a culture of which has been deposited in the Colección Española de Cultivos Tipo at the Universidad de Valencia, Spain, under the accession number CECT 20840. This deposit has been made under the provisions of the Budapest Treaty and all restrictions on the availability thereof to the public will be irrevocably maintained upon the granting of a patent on this application.

The organism was isolated from a soil sample collected in Spain.

### Colony characteristics

Colonies reach 4 cm diameter in ten days at 25°C on potato dextrose agar in a culture chamber that maintains a humidity of 42%. Colonies growing moderately, white to brownish violet, hairy in central area of the colony. It produces a yellow pigment at submerged hyphae.

### Microscopy & Identity

No sporulation nor conidiogenetic states could be observed. Sterile mycelia under those conditions. Taxonomical determination was achieved after a sequencing analysis of ITS1-5.8S-ITS2 ribosomal DNA region. Comparing the sequence obtained with the sequences deposited at Genebank databases, a 97% match was obtained compared with *Chaetomiaceae* and 96% match with *Chaetomium sp.*

Based on the preceding characteristics the culture has been determined as *Chaetomium sp.*

### Culture characteristics

The optimal temperature for growth in liquid media is 24-28°C. The pH range for growth is between 3.5 to 8.8. The best pH for production is 3.5-5.0. Growth was best with soy meal, glycerol and starch. Other carbon sources such as oatmeal, and dextrose can also be used.

*Chaetomium sp.* HT-GU-SUES-904 when cultured under controlled conditions in a suitable medium produces compound BMT9. This strain is grown in an aqueous nutrient medium, under aerobic and mesophilic conditions, preferably between 24 and 28°C at a pH 3.5 to 5.0.

A description of the process is as follows:

### Stock culture

A pure culture of *Chaetomium sp.* HT-GU-SUES-904 was kept frozen at -70°C in 20% glycerol.

### Preparation of inoculum

From a well grown agar culture, 8 plugs 1mm diameter were used to inoculate 40 ml of seed medium containing 2% oatmeal, 2% malt extract, 0.01% KH₂PO₄, 0.005% MgSO₄ and tap water in 250 ml shake flasks and cultured at 25°C on a rotary shaker at 200 rpm. The flasks were incubated for 72 hours in the dark and used as a first stage inoculum.

### Fermentation

250 ml of the same medium in 2 L Erlenmeyer flasks were inoculated with 6% from the first stage inoculum. The fermentation was carried for 7 days at 25°C on a rotary shaker at 200 rpm in the dark.

Production of this compound can be monitored by HPLC or any other method with enough sensitivity.

### Example 2: Production of BMT9, BMT9.2 and BMT9.5

Culture broth of fungus HT-GU-SUES-904 (7.5L) was stirred for 1h with 750 ml of Amberlite XAD-1180 and then filtered. The mycelial cake and the resin were extracted twice with 6L of a mixture of EtOAc/MeOH (3:1). The resultant suspension was filtered and partitioned between EtOAc and water. The organic layer was taken to dryness and the crude extract (19 g) was fractionated by VFC (Vacuum Flash Chromatography) on silica gel, eluted with a stepwise gradient of hexane/EtOAc/MeOH. Fractions containing BMT9 (eluted with hexane/EtOAc 1:1, 1.8 g) were applied to a silica gel column and flash-chromatographied by elution with a hexane/EtOAc gradient. Fractions containing BMT9 (eluted with hexane/EtOAc 8:2, 172 mg) were finally purified by reversed-phase column chromatography eluted with a stepwise gradient of acetone/water. Fractions eluted with acetone/water 75:25 afforded 115 mg of BMT9, 99% pure.

Fractions of the VFC containing BMT9.2 (eluted with hexane/EtOAc 2:8, 3.6 g) were applied to a silica gel column and flash-chromatographied by elution with a hexane/EtOAc gradient. Fractions eluted with hexane/EtOAc 4:6, afforded 375 mg of BMT9.2, 93% pure.

Compound BMT9.5 was obtained by acetylation of BMT9.2, dissolving BMT9.2 in pyridine and adding 1.2 equivalents of Ac₂O (acetic anhydride).

### BMT9 Structural Elucidation

Compound BMT9 is a colorless amorphous solid with 2 maximum absorptions in the UV spectrum at 223 and 268 nm. The compound was assigned the molecular formula C₂₆H₃₄N₆, based on HREIMS ([2M+Na]⁺ at m/z 907.4556), ¹³C-NMR and DEPT data. The ¹³C-NMR spectrum of BMT9 displayed a total of 26 carbon signals, which agree with the molecular formula obtained by HREIMS. These carbon resonances were classified as 5 methyl carbons, 6 methylene carbons (one of them olefinic), 5 methine carbons and 10 quaternary carbons (seven of them olefinic or aromatic and one carbonyl carbon) by DEPT experiments. Based on these data and the double bond equivalent for the molecular formula, BMT9 must contain five rings. gCOSY, gHSQC and gHMBC conducted on BMT9 permitted the corresponding proton and carbon NMR assignments to be made, and correlations were fully consistent with the 2D structure shown below.

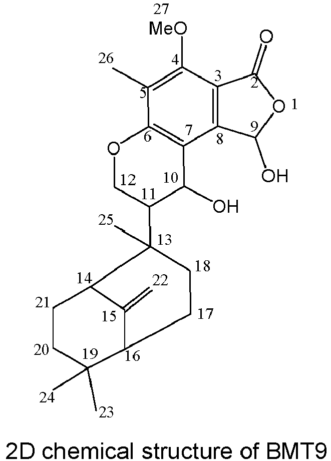

BMT9 spectroscopic data are shown in table 1.

**Table 1. ¹H and ¹³C NMR Spectral Data of BMT9 [δ (ppm), J_{HH} (Hz); CDCl₃]**

| Position | ¹³C (δ_{C}) | ¹H (δ_{H}) mult (J in Hz) | gCOSY | gHMBC |
|---|---|---|---|---|
| 1 | | | | |
| 2 | 167.7 | | | |
| 3 | 109.3 | | | |
| 4 | 156.5 | | | |
| 5 | 122.0 | | | |
| 6 | 159.3 | | | |
| 7 | 117.8 | | | |
| 8 | 145.7 | | | |
| 9 | 96.1 | 6.75 (1H, s) | | C2, C3, C7 |
| 10 | 61.1 | 5.08 (1H, broad s) | | C6, C11, C12 |
| 11 | 42.2 | 2.08 (1H, m) | 12 | C12, C13, C14, C18, C25 |
| 12 | 62.9 | 4.37 (2H, m) | 11 | C6, C10, C11, C13 |
| 13 | 38.7 | | | |
| 14 | 47.2 | 2.08 (1H, m) | 21 | 016, 020, 021, 025 |
| 15 | 151.5 | | | |
| 16 | 49.5 | 1.81(H, m) | 17 | 014, 015, 017, 018, 020, 022 |
| 17 | 25.1 | 1.70 (1H, m) 1.96 (1H, m) | 16, 18 | C13, C16,C18 |
| 18 | 31.7 | 1.68 (1H, m) 1.86 (1H, m) | 17 | C11, C13, C25 |
| 19 | 35.4 | | | |
| 20 | 34.7 | 1.14 (1H, broad d, 8.4 Hz) 1.70 (1H, m) | 21 | C14, C16, C19, C21, C23 |
| 21 | 25.8 | 1.70 (1H, m) 1.92 (1H, m) | 14, 20 | C15, C19 |
| 22 | 107.1 | 4.65 (1H, d, 2.3 Hz) 4.68 (1H, d, 2.3 Hz) | 22a, 22b | C14, C15, C16 |
| 23 | 29.0 | 0.92 (3H, s) | | C16, C19, C20, C24 |
| 24 | 28.7 | 0.98 (3H, s) | | C16, C19, C20, C23 |
| 25 | 21.7 | 1.11 (3H, s) | | C11, C13, C14, C18 |
| 26 | 8.65 | 2.05 (3H, s) | | C4, C5, C6 |
| 27 | 62.1 | 3.92 (3H, s) | | C4 |

### BMT9.2 Structural Elucidation

Compound BMT9.2 is a yellowish amorphous solid with 3 maximum absorptions in the UV spectrum at 250, 275 and 320 nm. The compound was assigned the molecular formula C₂₆H₃₂O₇, based on HREIMS ([M+H]+ at m/z 457.2228), ¹³C-NMR and DEPT data. The ¹³C-NMR spectrum of BMT9.2 displayed a total of 26 carbon signals, which agree with the molecular formula obtained by HREIMS. These carbon resonances were classified as 4 methyl carbons, 6 methylene carbons (one of them olefinic), 6 methine carbons (one of them an aldehyde) and 10 quaternary carbons (seven of them olefinic or aromatic and one carbonyl carbon) by DEPT experiments. Based on these data and the double bond equivalent for the molecular formula, BMT9.2 must contain five rings. gCOSY, gHSQC and gHMBC conducted on BMT9.2 allowed the corresponding proton and carbon NMR assignments to be made, and correlations were consistent with the 2D structure shown below.

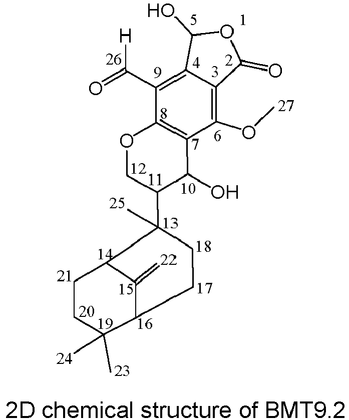

### BMT9.2 spectroscopic data are shown in table 2:

| Position | 13C (δC) | 1H (δH) mult (J in Hz) | gCOSY | gHMBC |
|---|---|---|---|---|
| 1 | | | | |
| 2 | 166 | | | |
| 3 | 108 | | | |
| 4 | 152 | | | |
| 5 | 96 | 6.79 (1H, s) | | C2, C3 |
| 6 | 162 | | | |
| 7 | 122 | | | |
| 8 | 163 | | | |
| 9 | 115 | | | |
| 10 | 59.8 | 5.18 (1H, s) | 11 | C6, C7, C8, C12 |
| 11 | 42.3 | 1.98 (1H, m) | 10, 12 | |
| 12 | 63.7 | 4.39 (2H, m) | 11 | C8, C10, C11 |
| 13 | 39.0 | | | |
| 14 | 47.4 | 2.05 (1H, m) | 21 | C15, C20, C22 |
| 15 | 151.3 | | | |
| 16 | 49.7 | 1.80 (1H, m) | 17 | C14, C15, C17, C22 |
| 17 | 26.0 | 1.74 (1H, m) | 16, 18 | |
| | | 1.90 (1H, m) | | |
| 18 | 31.9 | 1.75 (2H, m) | 17 | |
| 19 | 35.6 | | | |
| 20 | 34.9 | 1.18, (1H, m) | 21 | C14, C16, C19, C21 |
| | | 1.68 (1H, m) | | |
| 21 | 25.3 | 1.78 (1H, m) | 20 | |
| | | 2,00 (1H, m) | | |
| 22 | 107.7 | 4.67 (1H, d J=2.3) | | C14, C15, C16 |
| | | 4.70 (1H, d J=2.3) | | |
| 23 | 28.9 | 0.94 (3H, s) | | C16, C19, C20, C24 |
| 24 | 29.2 | 0.89 (3H, s) | | C16, C19, C20, C23 |
| 25 | 22.0 | 1.10 (3H, s) | | C11, C13, C14, C18 |
| 26 | 188 | 10.33 (1H, s) | | C4, C8, C9 |
| 27 | 64.1 | 3.40 (3H, s) | | C6 |

### Example 3: Generation of a reporter system capable of monitoring TAp73 transcription from the P1 promoter of the TP73 human gene.

The human *TP73* gene (**Figure 1A**) (from Horvat et al., 2021) generates different p73 isoforms from the P1 promoter located upstream of exon 1: the full length TAp73 and, due to alternative splicing, the N-terminally truncated isoforms ΔEx2p73, ΔEx2/3p73, and ΔN'p73.

A bimodal reporter vector, named P1-p73-Fluc-DsRed2, was engineered based on the previously published lentiviral system LV-CMV-Fluc-dsRed2 (Shah et al., 2008). In the original vector, which was shown to be fully functional in human NSCs as well as GB cells, luciferase and red fluorescent protein (DsRed) expression are regulated by the constitutive cytomegalovirus -CMV- promoter (Shah et al., 2008). Using this backbone, the CMV promoter was swapped for a 930 bp fragment of the human P1-*TP73* promoter contained in the P1-p73-Luc-pGL3-Basic vector (-859 to +71 of *TP73* gene) (Seelan et al., 2002) (**Figure 1A'**). To validate the generated system, we used the HCT116 p53-/- cells in which endogenous TAp73 expression can be induced by the genotoxic drug doxorubicin (Doxo), at RNA and protein levels (**Figure 1B** and Vossio et al., 2002, respectively). Next, we tested whether the engineered reporter vector could monitor these changes in endogenous TAp73 RNA levels. For this purpose, HCT116 p53-/- cells were either non-transfected (NT) or transfected with P1-p73-FlucDsRed vector and cultured with Doxo (black square fill pattern bar) or without treatment (grey bar) for the indicated time points (**Figure 1C**), at which the fluorescence levels (DsRed, Y axis) were measured by fluorimetry.

As depicted in **Figure 1C**, we detected an increase in endogenous P1-promoter activity after 24 and 48 hours of culture without treatment (grey bars). Moreover, even though Doxo treatment is associated to background autofluorescence (black bars), we detected an increase in reporter activity that was sustained after 48 hours (black square fill pattern bars). The data demonstrates that the generated construct is capable to serve as a reporter to monitor P1-*TP73* promoter activity, allowing the quantification of the endogenous P1-promoter driven expression, as well as the upregulation induced by drugs.

### Example 4: Screening of a small natural compound library to search for candidates capable of regulating P1-TP73 promoter activity in glioblastoma cells.

The conventional GB cell line U373 was used to perform a low-throughput chemical screen to identify P1-*TP73*-promoter regulators from a panel of 51 natural compounds with novel chemical structures (BMT-1 to BMT-51). Cells were seeded on black microplates and 24 h later they were transfected with 1 µg of P1-p73-Fluc-DsRed plasmid using Lipofectamine 3000^{™} reagent (ThermoFisher) following the manufacturer's instructions. Six hours later, transfected cells were treated either with 8 µg/ml of any of the tested compounds or with the drugs used as first line glioblastoma treatment: the methylating agent temozolomide (TMZ) and the chloroethyl-derivative of nitrosourea carmustine (BCNU), or equivalent volumes of DMSO. Cells were monitored by fluorescence microscopy and the reporter activity was quantified using a "*Synergy HT*" plate reader (Biotek) 48 h after treatment and normalized to fluorescence values of DMSO exposed cells (**Figure 2A**).

The compounds with a modulatory effect of a 2-fold fluorescence increase, (squares bars) or a 0.5-fold reduction (grey bars) were pre-selected (BMT-5, 6, 9, 18, 20, 30, 48 and 51) and their effect was validated using the same methodology (**Figure 2B**). Compounds 8 and 10, despite showing an increase in fluorescence, were discarded because of their autofluorescence. **The compound BMT9 had the strongest and most reproducible repressive effect over** P1-TP73-promoter **activity and thus, it was selected for further analysis.** The compound BMT18 was also found to have autofluorescence.

To demonstrate that BMT9 repressed P1-TP73-promoter activity in U373 cells, a quantitative analysis of the expression levels of the different p73 transcripts originated from the P1 promoter was performed under the same conditions as in the screening. Expression levels of TAp73 (**Figure 2C**), ΔEx2p73 (**Figure 2D**) and ΔEx2/3p73 (**Figure 2E**) were determined by qRT-qPCR using 18S ribosomal RNA as housekeeping control, and all the expression values were normalized to TAp73 expression in non-treated cells (NT). The most prominently expressed isoforms were TAp73 and ΔEx2/3p73, but BMT9 treatment reduced them all. ΔN'p73 was barely detected (Data not shown). The amino-truncated isoforms that are tumor-specific are vigorously expressed in advanced stages across a variety of highly aggressive human cancers (Knoll et al., 2011; Steder et al., 2013; George et al., 2015; Meier et al., 2016) and promote tumor initiation (Tannapfel et al., 2008) and cancer progression (Engelmann et al., 2015). Moreover, there is a somatic genomic rearrangement of *TP73* that generates an oncogenic Ex2/3p73 in small cell lung cancer (George et al., 2015). BMT9 represses the activity of the P1-*TP73* promoter, therefore it would be expected to downregulate the expression of any of the isoforms generated from this promoter in a given cell.

Next, we compared the effect of BMT9 and TMZ or BCNU treatments on endogenous TAp73 protein levels in U373 and T98G glioblastoma cell lines (**Figure 2F** **and G**). Cells were treated for 72 h either with BMT9 at 9 and 18 µM, or with TMZ and BCNU at the standard concentrations referred in other articles (350 µM and 150 µM, respectively). Protein extracts were obtained and analyzed by Western blot using a TAp73 specific antibody and GAPDH as a loading control. Densitometric quantification of the obtained blots are represented in **Figure 2F** **and G** (Y axis, ratio of TAp73/GAPDH band intensity and X axis, indicated treatments). These data showed that **BMT9, but not DMSO or other drugs (TMZ or BCNU) reduced the expression of P1-driven p73 isoforms and, in particular, it efficiently reduced endogenous TAp73 expression in conventional glioblastoma cells.**

### Example 5: BMT9 reduces P1-promoter driven isoforms, TAp73 in particular, in glioblastoma neural stem cell lines G144 and G179.

Cumulative evidence supports the idea of the existence of a small subpopulation of glioma neural stem-like cells (GNSCs), which are responsible, at least in part, for the initiation, progression, heterogeneity and increased resistance to treatments of glioblastoma (Hemmati et al., 2003; Singh et al., 2003; Bao et al., 2006). We were interested in identifying small molecules that preferentially affected GNSCs. Thus, we sought to study the effect of the identified compound on the pro-neural glioblastoma stem cell line G144. These cells, derived from human malignant gliomas, display stem cell properties: they have self-renewal, maintain the expression of the neural stem cell marker CD133 and can initiate high-grade gliomas following xenotransplantation, proving to be a useful tool for the study of agents that selectively target GNSCs (Pollard et al., 2009; Danovi et al., 2013).

qRT-PCR analysis showed that treatment with BMT9 for 72 h in G144 cells downregulated the transcript levels originated from the P1-TP73 promoter (TAp73, ΔEx2p73 and ΔEx2/3p73) in a dose dependent manner as indicated. As before, all isoforms were reduced by treatment, with TAp73 and ΔEx2/3p73 being the predominant isoforms, while ΔN'p73 was barely detected (**Figure 4A**). Ribosomal RNA 18S was used as housekeeping control, and all expression levels were normalized to the TAp73 NT value.

To specifically address BMT9 effect on endogenous TAp73 protein levels, we performed a Western blot analysis using an anti-TA-specific antibody. Cells were treated for 72 h either with a low dose of BMT9 (2.25 µM), or with TMZ and BCNU at the standard concentrations used in other articles (350 µM and 150 µM, respectively). Protein extracts were obtained and analyzed by Western blot (**Figure 4B****-C**) and densitometric quantification of the autoradiographies was carried out. The ratio of TAp73/GAPDH band intensity was calculated and protein expression levels relative to non-treated (NT) cells were represented (Y axis). The data demonstrate that, as in traditional GB cell lines, BMT9, but not the DMSO-vehicle control or other drugs (TMZ or BCNU), reduced effectively TAp73 expression in glioblastoma stem cells.

### Example 6: BMT9 is more effective against glioma neural stem cells than the first line drugs Temozolomide (TMZ) and Carmustine (BCNU).

A dose-response analysis of BMT9 (concentrations ranging from 4.5 µM-18 µM), BCNU (from 150 µM-500 µM) and TMZ (350 µM-700 µM) was performed in two conventional glioblastoma cell lines (T98G, U373) and the GNS cell line G144 by MTT-based cytotoxicity assays (**Figure 5A****-D**). Cells were seeded on p96-well plates and treated with the different compounds for 72 h. Then, the MTT reagent (3-(4,5-Dimethyl-2-thiazolyl)-2,5-diphenyl-2H-tetrazolium bromide diluted in PBS to a concentration of 1mg/ml), was added to each well to a final concentration of 0.33mg/ml, and incubated for 3 h at 37°C. After that, the media was removed, and formazan crystals were solubilized with DMSO. Absorbance was measured at 560 nm using a "Synergy HT" plate reader (Biotek). In the graphs, the Y axis represents the percentage of cell viability relative to non-treated cells. The IC50, when reached are marked by a rectangle. DMSO treatment had no effect on cell viability. As shown in **Figure 5A****-C,** BMT9 IC50 was lower than BCNU IC50 for all the analyzed cell lines (compare >9.0 µM vs. 500 µM in T98G; 18.0 µM vs. 250 µM in U373 or <4.5 µM vs. 250 µM in G144). Moreover, BMT9 concentrations as low as 2.25 µM (G144) (**Figure 5E**) or 6.75 µM (G179) (**Figure 5F**) still reduced viability 72 h after treatment. **Altogether these data indicate that BMT9 is more effective against all GB cell lines tested than TMZ of BCNU and has specificity against GNSC.**

### Example 7: BMT9 analogues, BMT9-2 and BMT9-5, are also effective against GNSC.

The cytotoxicity of two BMT9 analogues synthetized by Instituto Biomar S.A. was evaluated by MTT assays. BMT9.2 and BMT9.5 were found to be also active against GNSCs with an IC50≤ ≤4.5 µM (**Figure 6**).

### Example 8: BMT9 treatment at IC50 in GNSC results in a moderate induction of apoptosis and significantly reduces stemness.

To elucidate the mode of action of BMT9 in GNSC, we analyzed the effect of this compound at IC50 concentration (4.5 µM) in G144 cells. For apoptosis induction analysis, the cells were treated with increasing amounts of BMT9 (4.5 µM and 9 µM) and collected 48 h after treatment. Anexin V and propidium iodide staining was performed using the FITC Annexin V Apoptosis Detection Kit I (BD Biosciences), following the manufacturer's instructions. Flow cytometry was performed with the "MACSQuantify Analyzer" (Miltenyi Biotec) and data were analyzed with "MACSQuantify" software (Miltenyi Biotec) **(****Figure 7A****). These experiments indicated that while IC50 dosage of BMT9 only produced moderate levels of apoptosis, at higher doses (9 µM) the treatment strongly induced cell death.**

Next, we asked if BMT9 treatment at IC50 could specifically affect the stemness of GNSC. To address this point, cells were seeded and treated 24 h later with BMT9 (4.5-9 µM), TMZ (500 µM) or BCNU (250 µM). After 48 hours of treatment, cells were collected and stained with a CD133 (stemness marker) antibody (AC133, Milyenty Biotec) or the corresponding Ig-isotype control. Flow cytometric analysis were performed with the previously mentioned equipment. As shown in **Figure 7B**-C, BMT9 treatment, but not **TMZ or BCNU, significantly reduced CD133 expression levels (B, Mean Fluorescence Intensity, Y axis) and the percentage of CD133 positive cells within the treated population of G144 cells (C), in a dose response manner.**

To further investigate whether BMT9 reduced stemness potential of the GNSCs, we performed a sphere-forming clonogenic assay. Cells were seeded on sphere forming conditions, that is 40,000 cells/ml without laminin coating. Cells were treated with BMT9 at 4.5 µM and 9 µM 24 h after seeding. BMT9 was able to reduce sphere generation in a dose dependent manner in these cultures, even at a concentration at which induction of cell death is very low (4.5 µM) (Data not shown).

Moreover, we asked whether BMT9, at IC50 concentration, induced differentiation of these GNSC. For these experiments, cells were seeded under proliferation conditions and treated with BMT9 at 4.5 µM for 72 h. Cells were fixed with 3.7% paraformaldehyde for 15 min and immunostained with Nestin, Tuj1 and GFAP specific antibodies. Samples were analyzed using a Zeiss LSM800 confocal microscopy. To quantify the percentage of neural (Tuj-1) (**Figure 7D**) and astrocytic (GFAP+) (**Figure 7E**) differentiated cells, positive cells for each marker were manually counted and compared with the total number of cells. Immunofluorescence analysis **showed that BMT9 treatment reduced the expression of the stem cell marker Nestin (Data not shown) and induced neural differentiation under proliferating culture conditions (****Figure 7D****-E).**

Finally, to elucidate the molecular basis of BMT9 effect on the stemness potential of the GNSCs, we compared the transcriptome of non-treated G144-GNSC cells with those after BMT9 treatment. The DESeq2 analysis identified 6163 differentially expressed genes (DEGs) with a p-adj<0.01. Comparison of these DEGs with published gene signatures of stemness in GBM and other cancers, indicated that BMT9 treatment downregulated many stemness related genes (Table 3). Among the found DEGs we found GSC markers like *PROM1*(CD133), *LGR5,* or *SOX1* and *SOX4*, as well as key regulators involved in maintenance of the cancer cell stemness state like SOX9 (Wang et al., 2018)(Aldaz et al., 2020), CDC20 (Gujar et al., 2016) or AURKA, known to govern self-renewal capacity in glioma-initiating cells via stabilization/activation of β-catenin/Wnt signaling (Xia et al., 2013) and proposed, together with CCNA2, and TPX2, as ideal drug targets in GBM (Du et al., 2020).

**Table 3. Correlation of GNSCs transcriptomic profiles after BMT9 treatment with published gene signatures of stemness.**

| **Gene expression signature** | **Stemness-related genes downregulated by BMT9** | ***** |
|---|---|---|
| (Shats et al., 2011). Consensus stemness ranking (CSR) signature upregulated in cancer stem cell-enriched samples | *ASPM, ATAD2, BIRC5, BUB1, BUB1B, CCNA2, CCNB1, CCNB2, CDC20, CDC7, CDKN3, CENPA, CENPF, CEP55, CKS2, DTL, ECT2, ESPL1, FBXO5, FEN1, FOXM1, GPSM2, HMMR, KIF11, KIF14, KIF15,KIF20A, KIF23, KIF2C, KIF4A, MAD2L1, MCAM, MCM4, MCM6, MELK, MSH2, NCAPG, NCAPH, NEK2, PAICS, PBK, PDSS1, PTTG1, PUS7, RACGAP1, RAD51, RAD51AP1, RCC1, RRM2, SKP2, SMC2, SMC4, TOP2A, TPX2, TRIP13, TTK, UBE2C, ZWINT* | 58/91 (63.74%) |
| (Yan et al., 2011)Genes overexpressed in CD133+ GBM cells | *ARHGAP11A, ASPM, BIRC5, BRCA1, CDCA2, CDKN3, CENPH, CENPK, CKAP2L, CKS2, DHFR, DIAPH3, DLGAP5, DTL, ECT2, FANCI, FBXO5, GGH, GINS2, GMNN, KIF11, KIF15, KIF2C, KIF4A, KNTC1, LIG1, MAD2L1, MCM2, MCM3, MELK, MND1, NCAPH, NDC80, NEK2, NMU, NUF2, PBK, PCNA, POLQ, PRIM1, PROM1, PTTG1, RAD51, RRM2, SMC2, TIMELESS, TM4SF1, TOP2A, TPX2, TRIP13, TROAP, TTK, TYMS, WDR34* | 54/89 (60.67%) |
| (Patel et al., 2014)Consensus set of genes differentially expressed in GBM stem-like cells | *AGT, ATP1B2, CALM1, DDX3X, FXYD6, GRIA2, HAS2, IDH1, MAGED1, MARCKS, METTL7B, NFIA, PIK3R3, PMP2, PTPRZ1, SEMA6D* | 16/52 (30.77%) |
| (Malta et al., 2018). "Stemness hallmark" gene set (healthy- and cancer stem cells) | *ABCG2, EPAS1, LGR5, NES, PROM1* | 5/21 (23.81%) |
| (Neftel et al., 2019) Stem and neural progenitor cell signatures identified in GBM samples | *ABAT, ASCL1, BCAN, DCX, DLL1, DLL3, ELMO1, FXYD6, HES6, IGFBPL1, LRRN1, MEST, MLLT11, NEU4, NXPH1, OLIG1, SEZ6L, SHD, SOX4, TNR, TUBA1A, TUBB2A* | 22/89 (24.72%) |
| (Du et al., 2020). Hub genes associated with GBM stemness | *AURKA, CCNA2, ECT2, NUF2, RBMX, RRM2, TPX2* | 7/16 (43.75%) |
| (Feng et al., 2021).Stemness-related genes upregulated in HNSCC and in other cancer types, including brain cancer | *BUB1, CDKN3, CKAP2L, DLGAP5, KIF14, KIF18A, KIF23, TTK* | 8/8 (100.00%) |

| | | |
|---|---|---|
| *Number of genes from the published dataset that are downregulated by the treatment. GBM: glioblastoma. HNSCC: Head and neck squamous cell carcinoma | | |

It is noteworthy that G144 cells proliferate very slowly and, at maintenance culture conditions, about 90% of the cells are in G1 phase of the cell cycle (data not shown). Proliferation rate analysis of these cells, using immunostaining against phosphorylated-Histone 3 (p-His-3) to label proliferating cells, and DAPI staining to identify cell nuclei, indicated that only 2% on the cells were at mitosis (data not shown). Moreover, BMT9 treatment (4.5 µM) for 72 h significantly reduced the percentage of cells in mitosis to a 0.1% (Data not shown), probably reflecting the previously shown induction of differentiation.

**Altogether, this data supports the idea that BMT9 reduces the self-renewal and stemness potential of GNSCs.**

### Example 9: BMT9 has specific cytotoxic effect against GNSCs.

Previous studies have reported that the molecular signature of the GNSC G144 cell line corresponds to a 'proneural' subtype of GB with NSC features (Pollard et al., 2009; Danovi et al., 2013). Thus, to test BMT9 specificity we compared cellular viability when treating CD133-positive cells (either GNSCs or normal human fetal neural stem cells-NSC) versus CD133-negative human embryonic fibroblast (HEFs) (**Figure 8A**). MTT-based cytotoxicity assay (**Figure 7B****-D**) were performed as previously described. In long term viability experiments, BMT9 treatment at low concentrations (1.125-4.5 µM), **affected CD133-positive NSC and GNSC, but no HEF, with a stronger effect against GNSC, indicating that there is a therapeutic window of treatment.**

### Example 10: CD133-positive-TMZ resistant clones remain sensitive to BMT9.

While the first line therapy of gliomas has proven its effectiveness and improved clinical outcomes, acquired chemoresistance to alkylating agents has been identified as a major cause of treatment failure (Sarkaria et al., 2008). In a murine model of GB, long-term treatment with subtoxic TMZ doses enriched the side population of cancer stem cells, increasing the tumor aggressiveness (Bleau et al., 2009). Thus, we generated TMZ-resistant G144-clones that were selected upon treatment with TMZ either at 500 µM or 700 µM for several months (R500 and R700, respectively). The obtained clones maintained CD133 stem marker expression (**Figure 9A**). We then analyzed BMT9 dose-dependent effect on cell viability, by MTT assay, comparing the resistant clones to parental cells. For comparative purposes, in addition to the known IC50 of BCNU (500 µM) we also included a concentration that was in closer range (20 µM) to those used for BMT9 (3.37-18 µM). The resistant clones showed an IC50 at 72 hours between 4.5-9 µM in G144 R500 and 3.37-4.5 µM in G144 R700, very similar to parental cells (**Figure 7C****-D**), altogether suggesting that BMT9 could be useful in previously treated GBM patients that have developed resistances.

### Example 11: BMT9 treatment decreases GNSC migration and invasiveness in vitro.

The maintenance of the stem cell identity of the GNSC is linked with their ability to interact with its niche through adhesion mechanisms (Niola et al., 2012, 2013). Hence, targeting these mechanisms which are essential for self-renewal, niche-driven GNSC maintenance, and tumor growth could facilitate the development of more effective GB therapies (Lathia et al., 2015). Thus, we investigated the effect of BMT9 on the transcriptional profile of a set of invasiveness-related genes, as well as in cell migration and invasion using the scratch assays and a 3D spheroid cell-invasion assays, respectively.

For expression analysis, G144 cells were treated with BMT9 (4.5 µM) and after 72 h mRNA was extracted and qRT-PCR was performed. Expression analysis of a set of invasiveness-related genes revealed that treatment with BMT9 led to a downregulation of all the analyzed genes (**Figure 10A**).

The effect of BMT9 in cell migration was evaluated using scratch assays, also known as wound healing assays. Cells were seeded in culture inserts for wound healing assays (Ibidi) at a cell density of 35,000 cells/cm²; 24 h later the insert was removed to create the wound, and fresh medium was added with the corresponding treatment. Microscopy images were acquired at the time of treatment and 24 h later, using a Leica DMI3000 phase contrast microscope. Analysis was performed using the ImageJ software (Schindelin et al., 2012) and the MRI wound healing tool plugin (Suarez-Arnedo et al., 2020). Cell-free area was measured at the two indicated time points and the wound closure percentage relative to the starting point was calculated for each well individually. This assay was performed in G144 and in G179 cells (**Figure 10** **B and C, respectively**). In both cases, treatment with 4.5µM BMT9 in short term cultures (24 h) significantly impaired cell migration *in vitro.*

A hallmark of glioblastoma cell infiltration through a 3D environment is the extension of long, thin polarized membrane extensions that explore and penetrate the surrounding environment. The 3D spheroid cell-invasion model consists in generating cell spheroids by suspension culture that are subsequently embedded in a 3D matrix. G144 cells were seeded on non-adherent conditions (without laminin coating) at a cell concentration of 40,000 cells/ml. After 8 days, spheres were embedded in a 2:1 mixture of Matrigel and growth media. Matrigel mix was let to polymerize at 37°C for 30 minutes, and then it was covered with growth media. Migration was monitored by phase-contrast microscopy for 24 h and the area of each sphere at the initial and final time points was measured using the *ImageJ* software, in order to calculate the ratio between both areas (**Figure 10D****, Y axis**). BMT9 treatment significantly impaired the invasion of cells within the matrix surrounding the spheres.

**Altogether, these results demonstrates that BMT9 treatment reduces GNSC stemness capacity, migration and, more interestingly, invasiveness.**

### Example 12: Maximum Tolerated Dose and Pharmacokinetics of BMT9.

The Maximum Tolerated Dose (MTD) and Pharmacokinetics (PK) of BMT9 were determined after intraperitoneal administration to female C57BL/6 (~25 g) mice.

C57BL/6 female mice (9-13 weeks in age) were administered intraperitoneally with a single dose of BMT9 at 35, 50, 75, 100 or 200 mg/Kg. Total of 3 animals were used for each independent serial sampling experiment. The compound was dissolved in an appropriate vehicle (Cremophor:EtOH:H20 1:1:8). Body weight, distress and survival were monitored 1, 2, 4 hours and 8 hours post-administration and every day during the whole observation period. The MTD was set on the dose below the first dose that caused severe loss of body weight (>20%) or death of one or more animals of a dose group seven days after treatment. The MTD of BMT9 was therefore set on about 140 mg/kg. The concentrations of BMT9 in plasma was determined at different times per duplicate using a previously validated analytical method. The compound was injected via i.p at 100 mg/kg. Total of 3 animals were used for each independent serial sampling experiment. Blood samples were collected at 5, 15, 30 min, 1, 4, 10, 24, and 48 hours from each animal via cardiac puncture; 500 µL withdrawn at each time point. 25 microliters of blood sample were then used for quantitative bioanalysis by HPLC-MS/MS. The fundamental pharmacokinetic parameters were obtained from the non-compartmental analysis using PK Solutions 2.0.2. The results are represented in **Figure 11** and show that the highest concentration in the plasma is 7611 ng/mL at 4 hours after injection and progressively declined until reaching the control levels at 24 hours post-administration.

### LITERATURE

Al-Hajj M., Wicha M.S., Benito-Hernandez A., Morrison S.J., & Clarke M.F. (2003) Prospective identification of tumorigenic breast cancer cells. Proceedings of the National Academy of Sciences of the United States of America, 100, 3983-8.
Aldaz P., Otaegi-Ugartemendia M., Saenz-Antoñanzas A., Garcia-Puga M., Moreno-Valladares M., Flores J.M., Gerovska D., Arauzo-Bravo M.J., Samprón N., Matheu A., & Carrasco-Garcia E. (2020) SOX9 promotes tumor progression through the axis BMI1-p21CIP. Scientific Reports, 10, 357.
Amelio I., Markert E.K., Rufini A., Antonov A. V, Sayan B.S., Tucci P., Agostini M., Mineo T.C., Levine A.J., & Melino G. (2014) p73 regulates serine biosynthesis in cancer. Oncogene, 33, 5039-5046.
Aponte P.M. & Caicedo A. (2017) Stemness in Cancer: Stem Cells, Cancer Stem Cells, and Their Microenvironment. Stem Cells International, 2017, 5619472.
Bao S., Wu Q., McLendon R.E., Hao Y., Shi Q., Hjelmeland A.B., Dewhirst M.W., Bigner D.D., & Rich J.N. (2006a) Glioma stem cells promote radioresistance by preferential activation of the DNA damage response. Nature, 444, 756-760.
Bao S., Wu Q., McLendon R.E., Hao Y., Shi Q., Hjelmeland A.B., Dewhirst M.W., Bigner D.D., & Rich J.N. (2006b) Glioma stem cells promote radioresistance by preferential activation of the DNA damage response. Nature 2006 444:7120, 444, 756-760.
Beeler J.S., Marshall C.B., Gonzalez-Ericsson P.I., Shaver T.M., Santos Guasch G.L., Lea S.T., Johnson K.N., Jin H., Venters B.J., Sanders M.E., & Pietenpol J.A. (2019) p73 regulates epidermal wound healing and induced keratinocyte programming. PloS one, 14, e0218458.
Bleau A.-M., Hambardzumyan D., Ozawa T., Fomchenko E.I., Huse J.T., Brennan C.W., & Holland E.C. (2009) PTEN/PI3K/Akt pathway regulates the side population phenotype and ABCG2 activity in glioma tumor stem-like cells. Cell stem cell, 4, 226-235.
Cheng C., Feng S., Jiao J., Huang W., Huang J., Wang L., Jiang W., Jiang C., Dai M., Li Z., Zhang R., Sun J., & Shao J. (2018) DLC2 inhibits development of glioma through regulating the expression ratio of TAp73α/TAp73β. American journal of cancer research, 8, 1200-1213.
Costanzo A., Pediconi N., Narcisi A., Guerrieri F., Belloni L., Fausti F., Botti E., & Levrero M. (2014) TP63 and TP73 in cancer, an unresolved "family" puzzle of complexity, redundancy and hierarchy. FEBS Letters, 588, 2590-2599.
Danovi D., Folarin A., Gogolok S., Ender C., Elbatsh A.M.O., Engström P.G., Stricker S.H., Gagrica S., Georgian A., Yu D., U K.P., Harvey K.J., Ferretti P., Paddison P.J., Preston J.E., Abbott N.J., Bertone P., Smith A., & Pollard S.M. (2013) A high-content small molecule screen identifies sensitivity of glioblastoma stem cells to inhibition of polo-like kinase 1. PloS one, 8, e77053.
DeBerardinis R.J. & Cheng T. (2010) Q's next: the diverse functions of glutamine in metabolism, cell biology and cancer. Oncogene, 29, 313-324.
Du J., Yan X., Mi S., Li Y., Ji H., Hou K., Ma S., Ba Y., Zhou P., Chen L., Xie R., & Hu S. (2020) Identification of Prognostic Model and Biomarkers for Cancer Stem Cell Characteristics in Glioblastoma by Network Analysis of Multi-Omics Data and Stemness Indices . Frontiers in Cell and Developmental Biology , 8**,** .
Engelmann D., Meier C., Alia V., & Pützer B.M. (2015) A balancing act: orchestrating amino-truncated and full-length p73 variants as decisive factors in cancer progression. Oncogene, 34, 4287-4299.
Feng G., Xue F., He Y., Wang T., & Yuan H. (2021) The Identification of Stemness-Related Genes in the Risk of Head and Neck Squamous Cell Carcinoma. Frontiers in oncology, 11, 688545.
Fernandez-Garcia B., Vaqué J.P., Herreros-Villanueva M., Marques-Garcia F., Castrillo F., Fernandez-Medarde A., León J., & Marin M.C. (2007) p73 cooperates with Ras in the activation of MAP kinase signaling cascade. Cell Death & Differentiation, 14, 254-265.
Galli R., Binda E., Orfanelli U., Cipelletti B., Gritti A., De Vitis S., Fiocco R., Foroni C., Dimeco F., & Vescovi A. (2004) Isolation and characterization of tumorigenic, stem-like neural precursors from human glioblastoma. Cancer research, 64, 7011-7021.
George J., Lim J.S., Jang S.J., Cun Y., Ozretić L., Kong G., Leenders F., Lu X., Fernández-Cuesta L., Bosco G., Müller C., Dahmen I., Jahchan N.S., Park K.-S., Yang D., Karnezis A.N., Vaka D., Torres A., Wang M.S., Korbel J.O., Menon R., Chun S.-M., Kim D., Wilkerson M., Hayes N., Engelmann D., Pützer B., Bos M., Michels S., Vlasic I., Seidel D., Pinther B., Schaub P., Becker C., Altmüller J., Yokota J., Kohno T., Iwakawa R., Tsuta K., Noguchi M., Muley T., Hoffmann H., Schnabel P.A., Petersen I., Chen Y., Soltermann A., Tischler V., Choi C., Kim Y.-H., Massion P.P., Zou Y., Jovanovic D., Kontic M., Wright G.M., Russell P.A., Solomon B., Koch I., Lindner M., Muscarella L.A., la Torre A., Field J.K., Jakopovic M., Knezevic J., Castaños-Vélez E., Roz L., Pastorino U., Brustugun O.-T., Lund-Iversen M., Thunnissen E., Köhler J., Schuler M., Botling J., Sandelin M., Sanchez-Cespedes M., Salvesen H.B., Achter V., Lang U., Bogus M., Schneider P.M., Zander T., Ansén S., Hallek M., Wolf J., Vingron M., Yatabe Y., Travis W.D., Nürnberg P., Reinhardt C., Perner S., Heukamp L., Büttner R., Haas S.A., Brambilla E., Peifer M., Sage J., & Thomas R.K. (2015) Comprehensive genomic profiles of small cell lung cancer. Nature, 524, 47-53.
Gonzalez-Cano L., Fuertes-Alvarez S., Robledinos-Anton N., Bizy A., Villena-Cortes A., Fariñas I., Marques M.M., & Marin M.C. (2016) p73 is required for ependymal cell maturation and neurogenic SVZ cytoarchitecture. Developmental Neurobiology, 76, 730-747.
Gonzalez-Cano L., Herreros-Villanueva M., Fernandez-Alonso R., Ayuso-Sacido a, Meyer G., Garcia-Verdugo J.M., Silva a, Marques M.M., & Marin M.C. (2010) P73 Deficiency Results in Impaired Self Renewal and Premature Neuronal Differentiation of Mouse Neural Progenitors Independently of P53. Cell death & disease, 1, e109.
Gujar A.D., Yano H., & Kim A.H. (2016) The CDC20-APC/SOX2 signaling axis: An achilles' heel for glioblastoma. Molecular & Cellular Oncology, 3, e1075644.
Hemmati H.D., Nakano I., Lazareff J.A., Masterman-Smith M., Geschwind D.H., Bronner-Fraser M., & Kornblum H.I. (2003) Cancerous stem cells can arise from pediatric brain tumors. Proceedings of the National Academy of Sciences of the United States of America, 100, 15178-15183.
Horvat A., Tadijan A., Vlasić I., & Slade N. (2021) p53/p73 Protein Network in Colorectal Cancer and Other Human Malignancies. Cancers, 13, 2885.
Iscan E., Karakülah G., Ekin U., Ozturk M., Uzuner H., & Suner A. (2022) TAp73α is Upregulated in the Most Common Human Cancers. Molecular Biology, 56, 251-256.
Iwadate Y. (2016) Epithelial-mesenchymal transition in glioblastoma progression. Oncology letters, 11, 1615-1620.
Jiang P., Du W., & Yang X. (2013) A critical role of glucose-6-phosphate dehydrogenase in TAp73-mediated cell proliferation. Cell cycle (Georgetown, Tex.), 12, 3720-3726.
Kang M.J., Park B.J., Byun D.S., Park J.I., Kim H.J., Park J.H., & Chi S.G. (2000) Loss of imprinting and elevated expression of wild-type p73 in human gastric adenocarcinoma. Clinical cancer research: an official journal of the American Association for Cancer Research, 6, 1767-1771.
Klein A.M., de Queiroz R.M., Venkatesh D., & Prives C. (2021) The roles and regulation of MDM2 and MDMX: it is not just about p53. Genes & development, 35, 575-601.
Knoll S., Fürst K., Thomas S., Villanueva Baselga S., Stoll A., Schaefer S., & Pützer B.M. (2011) Dissection of cell context-dependent interactions between HBx and p53 family members in regulation of apoptosis: A role for HBV-induced HCC. Cell Cycle, 10, 3554-3565.
Landré V., Antonov A., Knight R., & Melino G. (2016) p73 promotes glioblastoma cell invasion by directly activating POSTN (periostin) expression. Oncotarget, 7, 11785-11802.
Lapidot T., Sirard C., Vormoor J., Murdoch B., Hoang T., Caceres-Cortes J., Minden M., Paterson B., Caligiuri M.A., & Dick J.E. (1994) A cell initiating human acute myeloid leukaemia after transplantation into SCID mice. Nature, 367, 645-648.
Lathia J.D., Gallagher J., Heddleston J.M., Wang J., Eyler C.E., Macswords J., Wu Q., Vasanji A., McLendon R.E., Hjelmeland A.B., & Rich J.N. (2010) Integrin alpha 6 regulates glioblastoma stem cells. Cell stem cell, 6, 421-432.
Lathia J.D., Mack S.C., Mulkearns-Hubert E.E., Valentim C.L.L., & Rich J.N. (2015) Cancer stem cells in glioblastoma. Genes & development, 29, 1203-1217.
Lee J., Kotliarova S., Kotliarov Y., Li A., Su Q., Donin N.M., Pastorino S., Purow B.W., Christopher N., Zhang W., Park J.K., & Fine H.A. (2006) Tumor stem cells derived from glioblastomas cultured in bFGF and EGF more closely mirror the phenotype and genotype of primary tumors than do serum-cultured cell lines. Cancer Cell, 9, 391-403.
López-Ferreras L., Martínez-García N., Maeso-Alonso L., Martin-Lopez M., Díez-Matilla Á., Villoch-Fernandez J., Alonso-Olivares H., Marques M.M., & Marin M.C. (2021) Deciphering the Nature of Trp73 Isoforms in Mouse Embryonic Stem Cell Models: Generation of Isoform-Specific Deficient Cell Lines Using the CRISPR/Cas9 Gene Editing System. Cancers, 13**,** .
Ma B., Zhang L., Zou Y., He R., Wu Q., Han C., & Zhang B. (2019) Reciprocal regulation of integrin β4 and KLF4 promotes gliomagenesis through maintaining cancer stem cell traits. Journal of Experimental and Clinical Cancer Research, 38, 1-17.
Maeso-Alonso L., López-Ferreras L., Marques M.M., & Marin M.C. (2021) p73 as a Tissue Architect. Frontiers in Cell and Developmental Biology, 9, 1954.
Malta T.M., Sokolov A., Gentles A.J., Burzykowski T., Poisson L., Weinstein J.N., Kamińska B., Huelsken J., Omberg L., Gevaert O., Colaprico A., Czerwińska P., Mazurek S., Mishra L., Heyn H., Krasnitz A., Godwin A.K., Lazar A.J., Stuart J.M., Hoadley K.A., Laird P.W., Noushmehr H., & Wiznerowicz M. (2018) Machine Learning Identifies Stemness Features Associated with Oncogenic Dedifferentiation. Cell, 173, 338-354.e15.
Meier C., Hardtstock P., Joost S., Alla V., & Pützer B.M. (2016) p73 and IGF1R Regulate Emergence of Aggressive Cancer Stem-like Features via miR-885-5p Control. Cancer Research, 76, 197-205.
Muppani N., Nyman U., & Joseph B. (2011) TAp73alpha protects small cell lung carcinoma cells from caspase-2 induced mitochondrial mediated apoptotic cell death. Oncotarget, 2, 1145-1154.
Napoli M. & Flores E.R. (2017) Another case for diet restriction: TAp73-expressing medulloblastomas are stunted by glutamine withdrawal. Genes & development, 31, 1715-1716.
Neftel C., Laffy J., Filbin M.G., Hara T., Shore M.E., Rahme G.J., Richman A.R., Silverbush D., Shaw M.L., Hebert C.M., Dewitt J., Gritsch S., Perez E.M., Gonzalez Castro L.N., Lan X., Druck N., Rodman C., Dionne D., Kaplan A., Bertalan M.S., Small J., Pelton K., Becker S., Bonal D., Nguyen Q.-D., Servis R.L., Fung J.M., Mylvaganam R., Mayr L., Gojo J., Haberler C., Geyeregger R., Czech T., Slavc I., Nahed B. V, Curry W.T., Carter B.S., Wakimoto H., Brastianos P.K., Batchelor T.T., Stemmer-Rachamimov A., Martinez-Lage M., Frosch M.P., Stamenkovic I., Riggi N., Rheinbay E., Monje M., Rozenblatt-Rosen O., Cahill D.P., Patel A.P., Hunter T., Verma I.M., Ligon K.L., Louis D.N., Regev A., Bernstein B.E., Tirosh I., & Suvà M.L. (2019) An Integrative Model of Cellular States, Plasticity, and Genetics for Glioblastoma. Cell, 178, 835-849.e21.
Niklison-Chirou M.V., Erngren I., Engskog M., Haglöf J., Picard D., Remke M., McPolin P.H.R., Selby M., Williamson D., Clifford S.C., Michod D., Hadjiandreou M., Arvidsson T., Pettersson C., Melino G., & Marino S. (2017) TAp73 is a marker of glutamine addiction in medulloblastoma. Genes and Development, 31, 1738-1753.
Niola F., Zhao X., Singh D., Castano A., Sullivan R., Lauria M., Nam H., Zhuang Y., Benezra R., Di Bernardo D., Iavarone A., & Lasorella A. (2012) Id proteins synchronize stemness and anchorage to the niche of neural stem cells. Nature Cell Biology, 14, 477-487.
Niola F., Zhao X., Singh D., Sullivan R., Castano A., Verrico A., Zoppoli P., Friedmann-Morvinski D., Sulman E., Barrett L., Zhuang Y., Verma I., Benezra R., Aldape K., Iavarone A., & Lasorella A. (2013) Mesenchymal high-grade glioma is maintained by the ID-RAP1 axis. The Journal of clinical investigation, 123, 405-417.
Niyazi M., Ghazizadeh M., Konishi H., Kawanami O., Sugisaki Y., & Araki T. (2003) Expression of p73 and c-Abl proteins in human ovarian carcinomas. Journal of Nippon Medical School = Nippon Ika Daigaku zasshi, 70, 234-242.
Novak U., Grob T.J., Baskaynak G., Peters U.R., Aebi S., Zwahlen D., Tschan M.P., Kreuzer K.A., Leibundgut E.O., Cajot J.F., Tobler A., & Fey M.F. (2001) Overexpression of the p73 gene is a novel finding in high-risk B-cell chronic lymphocytic leukemia. Annals of oncology: official journal of the European Society for Medical Oncology, 12, 981-986.
Patel A.P., Tirosh I., Trombetta J.J., Shalek A.K., Gillespie S.M., Wakimoto H., Cahill D.P., Nahed B. V, Curry W.T., Martuza R.L., Louis D.N., Rozenblatt-Rosen O., Suvà M.L., Regev A., & Bernstein B.E. (2014) Single-cell RNA-seq highlights intratumoral heterogeneity in primary glioblastoma. Science (New York, N.Y.), 344, 1396-1401.
Pollard S.M., Yoshikawa K., Clarke I.D., Danovi D., Stricker S., Russell R., Bayani J., Head R., Lee M., Bernstein M., Squire J.A., Smith A., & Dirks P. (2009) Glioma stem cell lines expanded in adherent culture have tumor-specific phenotypes and are suitable for chemical and genetic screens. Cell stem cell, 4, 568-580.
Pozniak C.D., Barnabé-Heider F., Rymar V. V, Lee A.F., Sadikot A.F., & Miller F.D. (2002) p73 is required for survival and maintenance of CNS neurons. The Journal of neuroscience : the official journal of the Society for Neuroscience, 22, 9800-9809.
Rozenberg J.M., Zvereva S., Dalina A., Blatov I., Zubarev I., Luppov D., Bessmertnyi A., Romanishin A., Alsoulaiman L., Kumeiko V., Kagansky A., Melino G., & Barlev N.A. (2021) Dual Role of p73 in Cancer Microenvironment and DNA Damage Response. Cells , 10**,** .
Santos Guasch G.L., Beeler J.S., Marshall C.B., Shaver T.M., Sheng Q., Johnson K.N., Boyd K.L., Venters B.J., Cook R.S., & Pietenpol J.A. (2018) p73 Is Required for Ovarian Follicle Development and Regulates a Gene Network Involved in Cell-to-Cell Adhesion. iScience, 8, 236-249.
Sarkaria J.N., Kitange G.J., James C.D., Plummer R., Calvert H., Weller M., & Wick W. (2008) Mechanisms of chemoresistance to alkylating agents in malignant glioma. Clinical cancer research: an official journal of the American Association for Cancer Research, 14, 2900-2908.
Schindelin J., Arganda-Carreras I., Frise E., Kaynig V., Longair M., Pietzsch T., Preibisch S., Rueden C., Saalfeld S., Schmid B., Tinevez J.-Y., White D.J., Hartenstein V., Eliceiri K., Tomancak P., & Cardona A. (2012) Fiji: an open-source platform for biological-image analysis. Nature Methods, 9, 676-682.
Seelan R.S., Irwin M., van der Stoop P., Qian C., Kaelin W.G.J., & Liu W. (2002) The human p73 promoter: characterization and identification of functional E2F binding sites. Neoplasia (New York, N. Y.), 4, 195-203.
Seymour T., Nowak A., & Kakulas F. (2015) Targeting Aggressive Cancer Stem Cells in Glioblastoma. Frontiers in oncology, 5, 159.
Shah K., Hingtgen S., Kasmieh R., Figueiredo J.L., Garcia-Garcia E., Martinez-Serrano A., Breakefield X., & Weissleder R. (2008) Bimodal Viral Vectors and &It;em&;In Vivo&It;/em> Imaging Reveal the Fate of Human Neural Stem Cells in Experimental Glioma Model. The Journal of Neuroscience, 28, 4406 LP - 4413.
Sharif T., Dai C., Martell E., Ghassemi-Rad M.S., Hanes M.R., Murphy P.J., Kennedy B.E., Venugopal C., Subapanditha M., Giacomantonio C.A., Marcato P., Singh S.K., & Gujar S. (2019) TAp73 Modifies Metabolism and Positively Regulates Growth of Cancer Stem-Like Cells in a Redox-Sensitive Manner. Clinical cancer research: an official journal of the American Association for Cancer Research, 25, 2001-2017.
Shats I., Gatza M.L., Chang J.T., Mori S., Wang J., Rich J., & Nevins J.R. (2011) Using a stem cell-based signature to guide therapeutic selection in cancer. Cancer research, 71, 1772-1780.
Singh S.K., Clarke I.D., Terasaki M., Bonn V.E., Hawkins C., Squire J., & Dirks P.B. (2003a) Identification of a cancer stem cell in human brain tumors. Cancer research, 63, 5821-5828.
Singh S.K., Clarke I.D., Terasaki M., Bonn V.E., Hawkins C., Squire J., & Dirks P.B. (2003b) Identification of a cancer stem cell in human brain tumors. Cancer Res., 63, 5821-5828.
Steder M., Alia V., Meier C., Spitschak A., Pahnke J., Fürst K., Kowtharapu B.S., Engelmann D., Petigk J., Egberts F., Schäd-Trcka S.G., Gross G., Nettelbeck D.M., Niemetz A., & Pützer B.M. (2013) DNp73 Exerts Function in Metastasis Initiation by Disconnecting the Inhibitory Role of EPLIN on IGF1R-AKT/STAT3 Signaling. Cancer Cell, 24, 512-527.
Still E.R. & Yuneva M.O. (2017) Hopefully devoted to Q: targeting glutamine addiction in cancer. British journal of cancer, 116, 1375-1381.
Stupp R., Mason W.P., van den Bent M.J., Weller M., Fisher B., Taphoorn M.J.B., Belanger K., Brandes A.A., Marosi C., Bogdahn U., Curschmann J., Janzer R.C., Ludwin S.K., Gorlia T., Allgeier A., Lacombe D., Cairncross J.G., Eisenhauer E., & Mirimanoff R.O. (2005) Radiotherapy plus concomitant and adjuvant temozolomide for glioblastoma. The New England journal of medicine, 352, 987-996.
Suarez-Arnedo A., Torres Figueroa F., Clavijo C., Arbeláez P., Cruz J.C., & Muñoz-Camargo C. (2020) An image J plugin for the high throughput image analysis of in vitro scratch wound healing assays. PloS one, 15, e0232565.
Subramanian D., Bunjobpol W., & Sabapathy K. (2015) Interplay between TAp73 Protein and Selected Activator Protein-1 (AP-1) Family Members Promotes AP-1 Target Gene Activation and Cellular Growth. The Journal of biological chemistry, 290, 18636-18649.
Sun Y., Pollard S., Conti L., Toselli M., Biella G., Parkin G., Willatt L., Falk A., Cattaneo E., & Smith A. (2008) Long-term tripotent differentiation capacity of human neural stem (NS) cells in adherent culture. Molecular and cellular neurosciences, 38, 245-258.
Tannapfel A., John K., Mise N., Schmidt A., Buhlmann S., Ibrahim S.M., & Pützer B.M. (2008) Autonomous growth and hepatocarcinogenesis in transgenic mice expressing the p53 family inhibitor DNp73. Carcinogenesis, 29, 211-218.
Tomasini R., Tsuchihara K., Wilhelm M., Fujitani M., Rufini A., Cheung C.C., Khan F., Itie-Youten A., Wakeham A., Tsao M.-S., lovanna J.L., Squire J., Jurisica I., Kaplan D., Melino G., Jurisicova A., & Mak T.W. (2008) TAp73 knockout shows genomic instability with infertility and tumor suppressor functions. Genes & development, 22, 2677-2691.
Ueda Y., Hijikata M., Takagi S., Chiba T., & Shimotohno K. (1999) New p73 variants with altered C-terminal structures have varied transcriptional activities. Oncogene, 18, 4993-4998.
Ugur H., Sayan A.E., Ozdamar S.O., Kanpolat Y., & Ozturk M. (2004) Expression of TAP73 and DeltaNP73 in malignant gliomas. Oncology reports, 11, 1337-1341.
Velletri T., Romeo F., Tucci P., Peschiaroli A., Annicchiarico-Petruzzelli M., Niklison-Chirou M., Amelio I., Knight R., Mak T., Melino G., & Agostini M. (2013) GLS2 is transcriptionally regulated by p73 and contributes to neuronal differentiation. Cell Cycle, 12, 3564-3573.
Vikhreva P., Melino G., & Amelio I. (2018) p73 Alternative Splicing: Exploring a Biological Role for the C-Terminal Isoforms. Journal of molecular biology, 430, 1829-1838.
Vossio S., Palescandolo E., Pediconi N., Moretti F., Balsano C., Levrero M., & Costanzo A. (2002) DN-p73 is activated after DNA damage in a p53-dependent manner to regulate p53-induced cell cycle arrest. Oncogene, 21, 3796-3803.
Wang X., Prager B.C., Wu Q., Kim L.J.Y., Gimple R.C., Shi Y., Yang K., Morton A.R., Zhou W., Zhu Z., Obara E.A.A., Miller T.E., Song A., Lai S., Hubert C.G., Jin X., Huang Z., Fang X., Dixit D., Tao W., Zhai K., Chen C., Dong Z., Zhang G., Dombrowski S.M., Hamerlik P., Mack S.C., Bao S., & Rich J.N. (2018) Reciprocal Signaling between Glioblastoma Stem Cells and Differentiated Tumor Cells Promotes Malignant Progression. Cell stem cell, 22, 514-528.e5.
Wang Z., Sturgis E.M., Guo W., Song X., Zhang F., Xu L., Wei Q., & Li G. (2012) Association of Combined p73 and p53 Genetic Variants with Tumor HPV16-Positive Oropharyngeal Cancer. PLOS ONE, 7, e35522.
Wei J., Zaika E., & Zaika A. (2012) p53 Family: Role of Protein Isoforms in Human Cancer. Journal of nucleic acids, 2012, 687359.
Wolfsberger J., Sakil H.A.M., Zhou L., van Bree N., Baldisseri E., de Souza Ferreira S., Zubillaga V., Stantic M., Fritz N., Hartman J., Rolny C., & Wilhelm M.T. (2021) TAp73 represses NF-κB-mediated recruitment of tumor-associated macrophages in breast cancer. Proceedings of the National Academy of Sciences, 118, e2017089118.
Xia Z., Wei P., Zhang H., Ding Z., Yang L., Huang Z., & Zhang N. (2013) AURKA Governs Self-Renewal Capacity in Glioma-Initiating Cells via Stabilization/Activation of β-catenin/Wnt Signaling. Molecular Cancer Research, 11, 1101-1111.
Xie N., Vikhreva P., Annicchiarico-Petruzzelli M., Amelio I., Barlev N., Knight R.A., & Melino G. (2018) Integrin-β4 is a novel transcriptional target of TAp73. Cell cycle (Georgetown, Tex.), 17, 589-594.
Yan X., Ma L., Yi D., Yoon J., Diercks A., Foltz G., Price N.D., Hood L.E., & Tian Q. (2011) A CD133-related gene expression signature identifies an aggressive glioblastoma subtype with excessive mutations. Proceedings of the National Academy of Sciences, 108, 1591-1596.
Yang A., Walker N., Bronson R., Kaghad M., Oosterwegel M., Bonnin J., Vagner C., Bonnet H., Dikkes P., Sharpe A., McKeon F., & Caput D. (2000) p73-deficient mice have neurological, pheromonal and inflammatory defects but lack spontaneous tumours. Nature, 404, 99-103.
Zitterbart K., Zavrelova I., Kadlecova J., Spesna R., Kratochvilova A., Pavelka Z., & Sterba J. (2007) p73 expression in medulloblastoma: TAp73/DeltaNp73 transcript detection and possible association of p73alpha/DeltaNp73 immunoreactivity with survival. Acta neuropathologica, 114, 641-650.

## Claims

1. Compound of general formula (I): 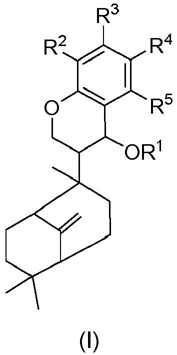 wherein
R¹ is hydrogen or a hydroxyl protecting group:
any two adjacent R², R³, R⁴, and R⁵, together with the atoms they attach to, form a 2,5-dihydrofuran ring which can be substituted with one or more substituents selected from the group consisting of =O and -OR⁷, wherein R⁷ is hydrogen or a hydroxyl protecting group; and
the other two R², R³, R⁴, or R⁵ are independently selected from the group consisting of hydrogen, C₁-C₆ alkyl, -CHO, -COOH and -OR⁶, wherein R⁶ is hydrogen or a hydroxyl protecting group;
or a pharmaceutically acceptable salt, stereoisomer or solvate thereof;
with the proviso that the compound is not

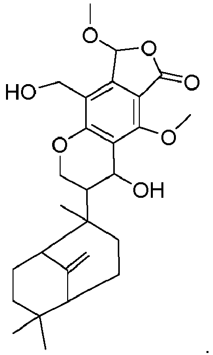

2. The compound according to claim 1, which is selected from a compound of general formula (Ia): 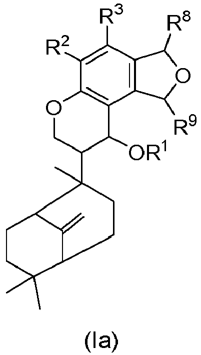 wherein
R¹ is hydrogen or a hydroxyl protecting group:
R² and R³ are independently selected from the group consisting of hydrogen, C₁-C₆ alkyl, -CHO, -COOH and -OR⁶, wherein R⁶ is hydrogen or a hydroxyl protecting group; and
R⁸ and R⁹ are independently selected from the group consisting of hydrogen, =O or -OR⁷, wherein R⁷ is hydrogen or a hydroxyl protecting group;
or a pharmaceutically acceptable salt, stereoisomer or solvate thereof.

3. The compound according to claim 1, which is selected from a compound of general formula (Ib): 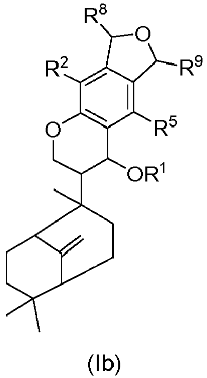 wherein
R¹ is hydrogen or a hydroxyl protecting group:
R² and R⁵ are independently selected from the group consisting of hydrogen, C₁-C₆ alkyl, -CHO, -COOH and -OR⁶, wherein R⁶ is hydrogen or a hydroxyl protecting group; and
R⁸ and R⁹ are independently selected from the group consisting of hydrogen, =O or -OR⁷, wherein R⁷ is hydrogen or a hydroxyl protecting group;
or a pharmaceutically acceptable salt, stereoisomer or solvate thereof.

4. The compound according to claim 1, which is selected from a compound of general formula (Ic): 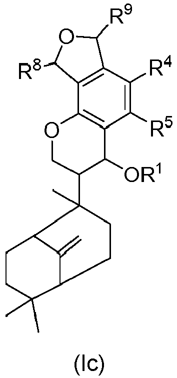 wherein
R¹ is hydrogen or a hydroxyl protecting group:
R⁴ and R⁵ are independently selected from the group consisting of hydrogen, C₁-C₆ alkyl, -CHO, -COOH and -OR⁶, wherein R⁶ is hydrogen or a hydroxyl protecting group; and
R⁸ and R⁹ are independently selected from the group consisting of hydrogen, =O or -OR⁷, wherein R⁷ is hydrogen or a hydroxyl protecting group
or a pharmaceutically acceptable salt, stereoisomer or solvate thereof.

5. The compound according to any one of claims 1 to 4, wherein each hydroxyl protecting group is independently selected from the group consisting of ethers, silyl ethers, esters, carbonates, carbamates, sulfonates, sulfinates and sulfonates.

6. The compound according to claim 1, which meets at least one of the following conditions:
- R¹ is hydrogen;
- at least one of R², R³, R⁴, and R⁵ is -OR⁶, wherein R⁶ is hydrogen or C₁-C₆ alkyl, preferably methyl;
- at least one of R², R³, R⁴, and R⁵ is C₁-C₆ alkyl, preferably methyl or -CH₂OH;
- at least one of R², R³, R⁴, and R⁵ is -CHO;
- at least one of R², R³, R⁴, and R⁵ is -COOR, wherein R is selected from H, C₁-C₆ alkyl, C₆-C₁₀ aryl and (C₆-C₁₀)aryl(C₁-C₆)alkyl;
- at least one of R⁸ and R⁹ is =O; and/or
- at least one of R⁸ and R⁹ is -OR⁷, wherein R⁷ is selected from hydrogen, C₁-C₆ alkyl, preferably methyl, and -COR', wherein R' is selected from C₁-C₆ alkyl, preferably methyl, C₆-C₁₀ aryl and (C₆-C₁₀)aryl(C₁-C₆)alkyl.

7. The compound according to claim 1, which is selected from the group consisting of: 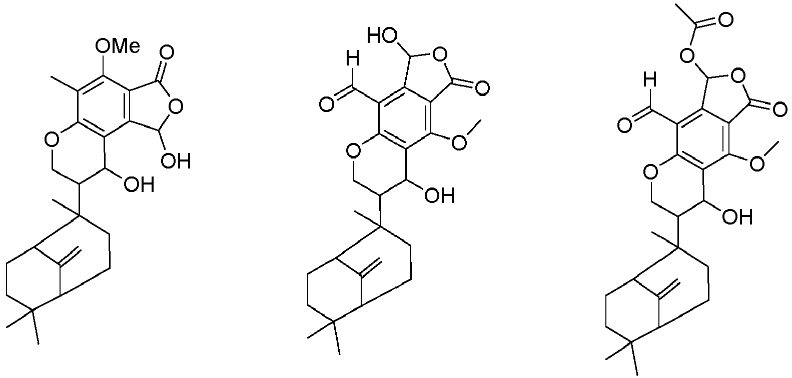 or a pharmaceutically acceptable salt, stereoisomer or solvate thereof.

8. A pharmaceutical composition comprising a compound as defined in any one of claims 1 to 7, or a pharmaceutically acceptable salt, stereoisomer or solvate thereof, and a pharmaceutically acceptable excipient.

9. The compound according to any one of claims 1 to 7 for use in medicine.

10. The compound according to any one of claims 1 to 7 for use in the treatment and/or prophylaxis of a disease associated with deregulation of p73 as well as stemness- and invasiveness-related genes.

11. The compound for use according to claim 10, wherein the disease is cancer, said cancer being preferably selected from the group consisting of cervical cancer(Wei et al., 2012), B-cell chronic lymphocytic leukemia, ovarian carcinomas, gastric adenocarcinoma, medulloblastoma, glioblastoma, glioma, astrocytoma, ependymoma, and oligodendroglioma breast invasive carcinoma, stomach adenocarcinoma, lung squamous cell carcinoma, colon adenocarcinoma, and esophageal carcinoma tumors..

12. The compound for use according to claim 11, wherein the cancer comprises Cancer Stem Cells, preferably CD133-CSC;
or wherein the cancer has its origin in CD133-positive neural stem cells (NSC) or neural progenitor cells (NPC).

13. The compound for use according to claim 11, in which the cancer is resistant to current chemotherapeutic treatments, and more particularly, resistant to the treatment of alkylating agents such as carmustine and temozolomide.

14. A process for preparing a compound as defined in any one of claims 1 to 7 which comprises
i) cultivating the fungal strain *Chaetomium sp.* HT-GU-SUES-904, available under accession number CECT 20840 from the Colección Española de Cultivos Tipo at Valencia University, Spain, in an aqueous nutrient medium, to obtain a natural compound as defined in any one of claims 1 to 7 in a culture broth and
ii) recovering said resulting natural compound from the culture broth;
or
i) cultivating the fungal strain *Chaetomium sp.* HT-GU-SUES-904, available under accession number CECT 20840 from the Colección Española de Cultivos Tipo at Valencia University, Spain, in an aqueous nutrient medium, to obtain a natural compound in a culture broth and
ii) either recovering the resulting natural compound from the culture broth and modifying it to obtain a compound as defined in any one of claims 1 to 7, or modifying the resulting natural compound to obtain a compound as defined in any one of claims 1 to 7 in a reaction crude and recovering said compound from the reaction crude.

15. Strain of *Chaetomium sp.* HT-GU-SUES-904, available under accession number CECT 20840 from the Colección Española de Cultivos Tipo at Valencia University.
